# EUROPEAN PATENT APPLICATION

(11) **EP 3 603 388 A1**
(43) Date of publication of application: **05.02.2020**
(21) Application number: 18776765.2
(22) Date of filing: 05.03.2018
(51) Int. Cl.: A01K 67/00, A01K 11/00, G06Q 50/02

(54) **BEHAVIOR SPECIFYING DEVICE, BEHAVIOR SPECIFYING METHOD AND PROGRAM**

(30) Priority: 31.03.2017 JP 2017070138; 02.10.2017 JP 2017192742
(71) Applicant: NTT Technocross Corporation, Minatu-ku, Tokyo 108-8202 (JP)
(72) Inventor: OYA, Hitomi, Tokyo 108-8202 (JP); ENDO, Ken, Tokyo 108-8202 (JP); HATANAKA, Masanori, Tokyo 108-8202 (JP)
(74) Representative: Vinsome, Rex Martin
(86) International application number: PCT/JP2018/008311
(87) International publication number: WO 2018/180242

(57) **Abstract**

A behavior identifying device that identifies a behavior of a farm animal includes a memory unit configured to store acceleration data measured by an acceleration sensor attached to the farm animal; an obtainer configured to obtain one or more acceleration data items for a predetermined period of time from among the acceleration data stored in the memory unit; an indicator calculator configured to calculate predetermined indicators from the one or more acceleration data items obtained by the obtainer; and an identifying unit configured to identify the behavior of the farm animal based on the indicators calculated by the indicator calculator and an identification model generated in advance for identifying the behavior of the farm animal.

## Description

### TECHNICAL FIELD

The present invention relates to a behavior identifying device, a behavior identifying method, and a program.

### BACKGROUND ART

The milking period of dairy cows starts after mating (artificial insemination) and giving birth to calves (after delivery), and this period is called the lactation period. It has been known that the lactation period lasts approximately 305 days. Also, in preparation for the next delivery, it is common to stop milking approximately 60 days before the next scheduled delivery date. This 60-day period is called the dry period. Dairy cows repeat delivery, a lactation period, and a dry period every year.

Dairy farmers who keep dairy cows are required to cross (artificial insemination) dairy cows within the dry period every year in order to continuously milk cows. In mating (artificial insemination) of dairy cows, it is necessary to discover the estrus of dairy cows. In order to find the estrus of dairy cows, it is practiced to confirm external signs of dairy cows or to confirm behaviors often seen during the estrus. Also, management of the estrus period based on breeding records using a ledger is also practiced.

### RELATED ART DOCUMENTS

### PATENT DOCUMENTS

Non-Patent Document 1: Ryo Abe, "Basic Agricultural Seminar: Basics of Farm Animal Breeding", New Edition, Rural Culture Association Japan, Incorporated Association, April 2008, p.109, p.122-124

### SUMMARY OF INVENTION

### [PROBLEM TO BE SOLVED BY THE INVENTION]

Here, for example, in large dairy farms that keep several thousand dairy cows or more, a heavy burden has been posed on dairy farmers to confirm the external signs and behaviors of dairy cows one by one. On the contrary, for example, if it is possible to identify behaviors of dairy cows being kept so as to easily find the behaviors often seen during the estrus, the burden posed on the dairy farmer can be alleviated.

Also, identifying the behaviors of dairy cows also enables not only to find the estrus, but also to find abnormal behaviors due to illness, injury, and the like, and to contribute to health care of the dairy cows.

One embodiment of the present invention has been made in view of the above points, and has an object to identify the behavior of a farm animal.

### [MEANS FOR SOLVING THE PROBLEM]

In order to solve the above problem, a behavior identifying device that identifies a behavior of a farm animal includes a memory unit configured to store acceleration data measured by an acceleration sensor attached to the farm animal; an obtainer configured to obtain one or more acceleration data items for a predetermined period of time from among the acceleration data stored in the memory unit; an indicator calculator configured to calculate predetermined indicators from the one or more acceleration data items obtained by the obtainer; and an identifying unit configured to identify the behavior of the farm animal based on the indicators calculated by the indicator calculator and an identification model generated in advance for identifying the behavior of the farm animal.

### [ADVANTAGE OF THE INVENTION]

It is possible to identify the behavior of a farm animal.

### BRIEF DESCRIPTION OF DRAWINGS

FIG. 1 is a diagram illustrating an example of an overall configuration of a behavior identifying system according to a first embodiment;
FIG. 2 is a diagram illustrating an example of behavior identification of a cow;
FIG. 3 is a diagram illustrating an example of measured data stored in a measured data memory unit according to the first embodiment;
FIG. 4 is a diagram illustrating an example of a behavior identifying model;
FIG. 5 is a diagram illustrating an example of a functional configuration of a behavior identifying processor according to the first embodiment;
FIG. 6 is a flow chart illustrating an example of a behavior identifying process according to the first embodiment;
FIG. 7 is a diagram illustrating an example of an overall configuration of a behavior identifying system according to a second embodiment;
FIG. 8 is a diagram illustrating an example in the case of identifying "standing" or "lying" by atmospheric pressure analysis;
FIG. 9 is a diagram illustrating an example of measured data stored in a measured data memory unit according to the second embodiment;
FIG. 10 is a diagram illustrating an example of a functional configuration of a behavior identifying processor according to the second embodiment;
FIG. 11 is a flow chart illustrating an example of a behavior identifying process for standing or lying behavior according to the second embodiment;
FIG. 12 is a diagram illustrating an example of a range for obtaining measured data and reference atmospheric pressure data according to a third embodiment;
FIG. 13 is a diagram illustrating an example of an overall configuration of a behavior identifying system according to a fourth embodiment;
FIG. 14 is a diagram illustrating an example of behavior identification of a cow;
FIG. 15 is a diagram illustrating an example of measured data stored in a measured data memory unit;
FIG. 16 is a diagram illustrating an example of a functional configuration of a behavior identifying processor according to the fourth embodiment;
FIG. 17 is a flow chart illustrating an example of a behavior identifying process by motion-activeness analysis;
FIG. 18 is a flow chart illustrating an example of a behavior identifying process by atmospheric pressure analysis;
FIG. 19 includes diagrams illustrating examples of behavior identification by atmospheric pressure analysis; and
FIG. 20 is a diagram illustrating an example in the case of identifying a behavior by atmospheric pressure analysis in a free-barn type cowshed.

### EMBODIMENTS OF THE INVENTION

In the following, embodiments of the present invention will be described with reference to the drawings. In the following, cases will be described in which behaviors of a cow, as an example of a farm animal, are identified. However, the farm animal is not limited to a cow.

### [First embodiment]

### <Overall configuration>

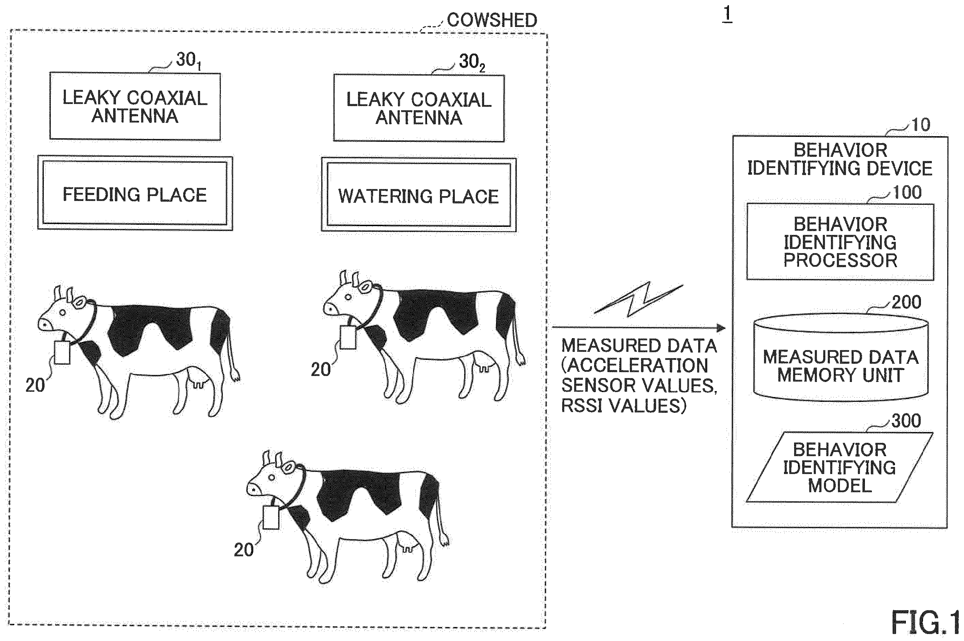

First, an overall configuration of a behavior identifying system 1 according to the present embodiment will be described with reference to FIG. 1. FIG. 1 is a diagram illustrating an example of an overall configuration of the behavior identifying system 1 according to the first embodiment.

As illustrated in FIG. 1, the behavior identifying system 1 according to the present embodiment includes a behavior identifying device 10 to identify the behavior of a cow, one or more tags 20 attached to cows, and one or more leaky coaxial antennas 30 to transmit a predetermined radio wave to the surroundings. Note that the tag 20 is favorably attached to be fixed to the neck portion of a cow.

The tag 20 is a device attached to a cow. One tag 20 is attached to one cow. The tag 20 includes an acceleration sensor that measures the acceleration of a cow (triaxial acceleration in X-axis, Y-axis, and Z-axis) to which the tag 20 is attached, and a radio wave sensor to measure an RSSI (Received Signal Strength Indicator) of a radio wave received from the leaky coaxial antenna 30.

The tag 20 transmits measured data including acceleration sensor values measured by the acceleration sensor and an RSSI value measured by the radio wave sensor to the behavior identifying device 10 every predetermined interval (e.g., every two seconds). The measured data transmitted to the behavior identifying device 10 is accumulated (stored) in a measured data memory unit 200, which will be described later. The measured data memory unit 200 stores multiple measured data items for each predetermined interval (e.g., every two seconds).

The leaky coaxial antenna 30 is an antenna that is installed in a predetermined place such as a feeding place and a watering place in a cowshed. The leaky coaxial antenna 30 transmits a predetermined radio wave to the surroundings through a leaky coaxial cable. Note that in the following, in the case of distinguishing a leaky coaxial antenna 30 installed in a feeding place from a leaky coaxial antenna 30 installed in a watering place, these are denoted as the "leaky coaxial antenna 301" and the "leaky coaxial antenna 302", respectively.

The behavior identifying device 10 is one or more computers that identify the behavior of a cow. The behavior identifying device 10 includes a behavior identifying processor 100, the measured data memory unit 200, and a behavior identifying model 300.

The behavior identifying processor 100 identifies the behavior of a cow based on measured data stored in the measured data memory unit 200 and the behavior identifying model 300. The behavior identifying processor 100 is implemented by a process which one or more programs installed in the behavior identifying device 10 cause a CPU (Central Processing Unit) or the like to execute.

The measured data memory unit 200 stores measured data received from the tag 20. The measured data memory unit 200 can be implemented by using an auxiliary storage device such as an HDD (Hard Disk Drive) or SSD (Solid State Drive).

The behavior identifying model 300 is a model for identifying a behavior from acceleration sensor values included in measured data. The behavior identifying model 300 is generated in advance by a method of machine learning such as SVM (Support Vector Machine), in accordance with, for example, the type of farm animal or the type of behavior to be identified. The behavior identifying model 300 is stored in an auxiliary storage device such as an HDD or SSD.

Note that the configuration of the behavior identifying system 1 illustrated in FIG. 1 is an example, and another configuration may be adopted. For example, the behavior identifying device 10 may be constituted with multiple computers. Also, for example, a device connected to the behavior identifying device 10 via a network (a cloud server, etc.) may have a part of the functions of the behavior identifying processor 100. Further, for example, instead of the tag 20, an acceleration sensor and a radio wave sensor may be separately attached to a cow.

### <Identification of behaviors of cow>

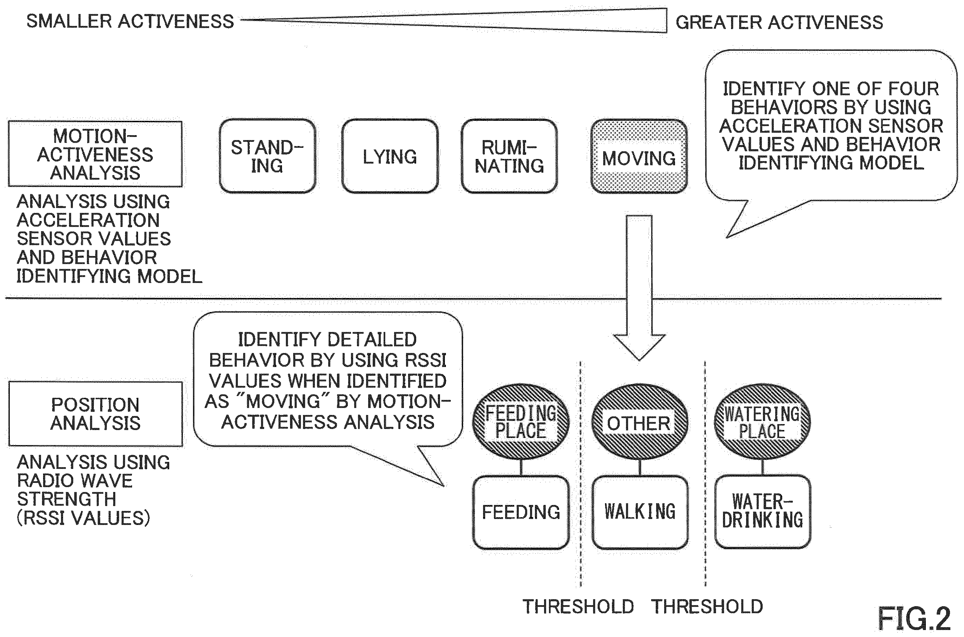

Here, behaviors of a cow to be identified by the behavior identifying system 1 according to the present embodiment, and an outline of an identification method will be described with reference to FIG. 2. FIG. 2 is a diagram illustrating an example of behavior identification of a cow.

First, assume that behaviors of a cow to be identified by the behavior identifying system 1 according to the present embodiment include four behaviors of "standing", "lying", "ruminating", and "moving" in ascending order of activeness of the motions. Note that the activeness of the behaviors of "standing" and "lying" may be reversed. In other words, the four behaviors may be arranged in the order of "lying", "standing", "ruminating", and "moving" in ascending order of activeness of the motions.

In addition, it is assumed that "moving" among these is further detailed into one of three behaviors of "feeding", "walking", and "water-drinking". Therefore, it is assumed that in the present embodiment, the behavior of a cow is identified as one of the six behaviors of "standing", "lying", "ruminating", "feeding", "walking", and "water-drinking".

The behavior of "standing" is a state of a cow that is standing. The behavior of "lying" is a state of a cow that is lying down. Note that these two behaviors of "standing" and "lying" are states in which a cow is not actively moving (i.e., the cow is standing still or the cow is lying still).

The behavior of "ruminating" is a state of a cow that is ruminating (a behavior of chewing food that has been once swallowed and returned to the mouth). The behavior of "moving" is a state of a cow that is performing one of the three behaviors of "feeding", "walking", and "water-drinking". The behavior of "feeding" is a state of a cow that is feeding itself with feed. The behavior of "walking" is a state of a cow that is walking. The behavior of "water-drinking" is a state of a cow that is drinking water.

Which behavior among "standing", "lying", "ruminating", and "moving" the cow is performing is identified by motion-activeness analysis. The motion-activeness analysis identifies the behavior of a cow among "standing", "lying", "ruminating", and "moving", based on acceleration sensor values included in measured data and the behavior identifying model 300. More specifically, the motion-activeness analysis calculates predetermined indicators from acceleration sensor values for a predetermined period of time (e.g., 10 minutes), where the indicators include a maximum value of swing of the L2 norms of the acceleration sensor values, and the standard deviation of the L2 norms of the acceleration sensor values during the predetermined period of time. Note that the maximum value of swing of the L2 norms is a maximum absolute value from among differences between the average value of the L2 norms of the acceleration sensor values for the predetermined period of time, and the values of the L2 norms of the acceleration sensor values for the predetermined period of time.

Then, the behavior is identified on the behavior identifying model 300 that classifies a behavior into one of four regions of "standing", "lying", "ruminating", and "moving", by determining in which of the four regions the calculated indicators are included.

Also, in the case where a behavior is identified as "moving" by the motion-activeness analysis, which of the three behaviors among "feeding", "walking", and "water-drinking" the cow is performing is identified by position analysis. The position analysis identifies the behavior of a cow as one of the three behaviors of "feeding", "walking", and "water-drinking", based on RSSI values included in measured data and a predetermined threshold value. More specifically, the position analysis identifies the behavior as "feeding" if the RSSI value of a radio wave received from the leaky coaxial antenna 301 installed in a feeding place exceeds a predetermined threshold value (i.e., when the cow is in the feeding place). Similarly, if the RSSI value of a radio wave received from the leaky coaxial antenna 302 installed in a watering place exceeds a predetermined threshold value (i.e., when the cow is in the watering place), the behavior is identified as "water-drinking". Meanwhile, if neither of the above conditions is satisfied (i.e., when the cow is not in the feeding place and not in the watering place), the behavior is identified as "walking".

Note that, for example, in the case where a GPS (Global Positioning System) receiver or the like is included in the tag 20, the position analysis may be performed using positional information obtained from the GPS receiver. In this case, from the positional information obtained from the tag 20, it is possible to locate whether the cow is in the feeding place, in the watering place, or not in the feeding place and not in the watering place.

As above, the behavior identifying system 1 according to the present embodiment identifies one of the four behaviors of "standing", "lying", "ruminating", and "moving" from acceleration sensor values received from the tag 20 attached to a cow. Also, in the case of identifying "moving" as the behavior of the cow, the behavior identifying system 1 according to the present embodiment further details the behavior into one of the three behaviors of "feeding", "walking", and "water-drinking" from RSSI values received from the tag 20 attached to the cow. This enables to identify the behavior of the cow as one of the six behaviors of "standing", "lying", "ruminating", "feeding", "walking", and "water-drinking".

Therefore, by displaying identified behaviors of cows as such, for example, on a display device (a display or the like) of the behavior identifying device 10, it is possible to easily confirm behaviors often observed during the estrus. Also, this also leads to early detection of abnormal behaviors due to illness or injury, and makes health care for the cows easier.

### <Measured data stored in measured data memory unit 200>

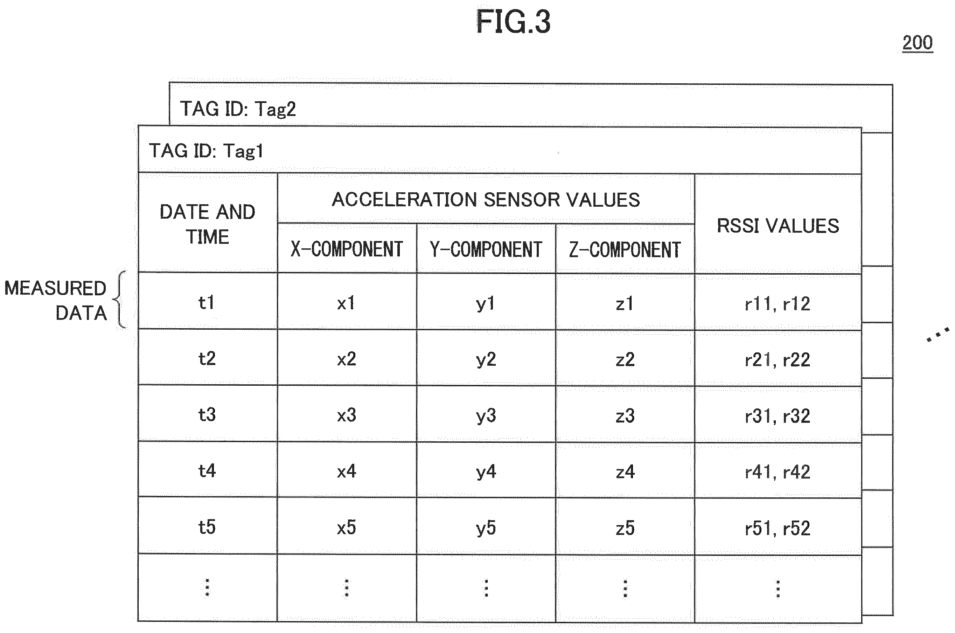

Here, measured data stored in the measured data memory unit 200 according to the present embodiment will be described with reference to FIG. 3. FIG. 3 is a diagram illustrating an example of measured data stored in the measured data memory unit 200 according to the first embodiment. Note that in the case of receiving measured data from the tag 20, the behavior identifying device 10 may cause the behavior identifying processor 100 to store (accumulate) the received measured data in the measured data memory unit 200.

As illustrated in FIG. 3, the measured data memory unit 200 stores one or more items of measured data for each tag ID that identifies a tag. Note that one tag 20 is attached to one cow; therefore, the tag ID may serve as information for identifying a cow (individual identification information for a cow) .

Measured data includes date and time, acceleration sensor values, and RSSI values. The date and time are, for example, a date and time when the tag 20 transmitted the measured data. Note that the date and time may be a date and time when the behavior identifying device 10 received the measured data.

The acceleration sensor values are acceleration values measured by an acceleration sensor included in the tag 20. The acceleration sensor values include an X-component representing an acceleration component in the X-axis direction, a Y-component representing an acceleration component in the Y-axis direction, and a Z-component representing an acceleration component in the Z-axis direction. For example, measured data at a date and time "t1" includes the X-component "x1", the Y-component "y1", and the Z-component "z1" as the acceleration sensor values.

The RSSI values are values of the RSSI measured by a radio wave sensor included in the tag 20. The RSSI values include, for example, an RSSI value of a radio wave received from the leaky coaxial antenna 301 installed in the feeding place, and an RSSI value of a radio wave received from the leaky coaxial antenna 302 installed in the watering place. For example, the measured data at the date and time "t1" includes an RSSI value "r11" received from the leaky coaxial antenna 301 installed in the feeding place, and an RSSI value "r12" received from the leaky coaxial antenna 302 installed in the watering place.

In this way, in the measured data stored in the measured data memory unit 200 according to the present embodiment, measured data including date and time, acceleration sensor values, and RSSI values is accumulated (stored) for each tag ID.

### <Behavior identifying model 300>

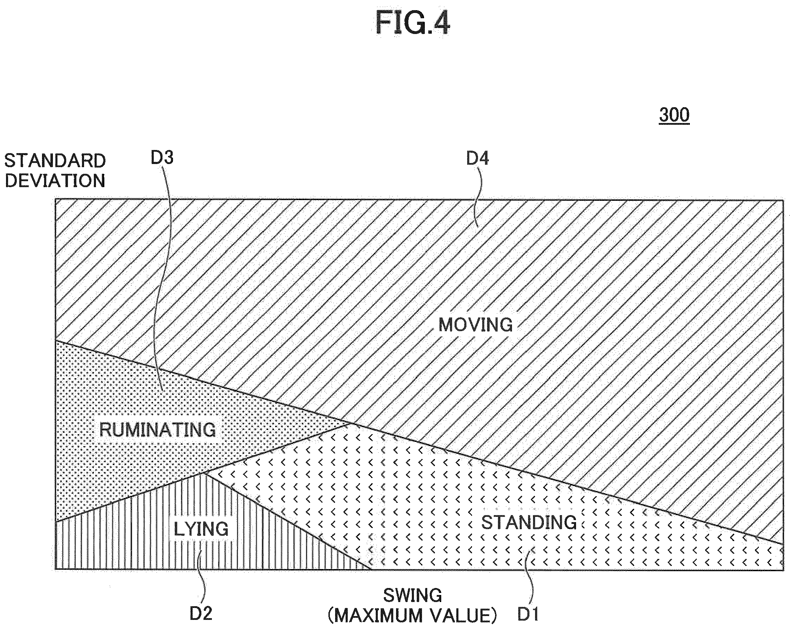

Here, the behavior identifying model 300 for identifying a behavior from acceleration sensor values included in measured data will be described with reference to FIG. 4. FIG. 4 is a diagram illustrating an example of the behavior identifying model 300.

As illustrated in FIG. 4, the behavior identifying model 300 is represented as a relationship graph that illustrates a relationship between the maximum value of swing of the L2 norms of acceleration sensor values for a predetermined period of time (e.g., 10 minutes) and the standard deviation of the L2 norms, where the horizontal axis represents the maximum value of swing and the vertical axis represents the standard deviation.

For example, if the maximum value of swing of the L2 norms of acceleration sensor values and the standard deviation for a certain 10 minutes fall in a region D1, the behavior of the cow for the 10 minutes is identified as "standing". Also, for example, if the maximum value of swing of the L2 norms of acceleration sensor values and the standard deviation for a certain 10 minutes fall in a region D2, the behavior of the cow for the 10 minutes is identified as "lying".

Similarly, for example, if the maximum value of swing of the L2 norms of acceleration sensor values and the standard deviation for a certain 10 minutes fall in a region D3, the behavior of the cow for the 10 minutes is identified as "ruminating". Note that the region D3 identified as "ruminating" may be further divided into a region where a cow is "ruminating" while the cow is in a state of standing (denoted as "ruminating while standing") and a region where a cow is "ruminating" while the cow in a state of lying (denoted as "ruminating while lying").

Also, similarly, for example, if the maximum value of swing of the L2 norms of acceleration sensor values and the standard deviation for a certain 10 minutes fall in a region D4, the behavior of the cow for the 10 minutes is identified as "moving".

Note that although the example illustrated in FIG. 4 illustrates a case where the regions D1 to D4 in the behavior identifying model 300 do not overlap each other, two or more of the regions D1 to D4 may partially overlap each other.

As such, the behavior identifying model 300 is a model in which regions are defined to represent a relationship between the maximum value and the standard deviation of the L2 norms of acceleration sensor values for a predetermined period of time (e.g., 10 minutes) and behaviors of a cow. Such a behavior identifying model 300 is generated in advance by a machine learning method such as SVM. Note that SVM is an example; and a model may be generated by various machine learning methods such as a neural network. Also, for example, even when a cow performs the same behavior, specific acceleration sensor values vary depending on the type of acceleration sensor. However, the positional relationship among the regions D1 to D4 representing the respective behaviors virtually remains unchanged.

Note that in the following, the maximum value of swing of the L2 norms may also simply referred to as the "maximum value of the L2 norms" or the "maximum value".

### <Functional configuration of behavior identifying processor 100>

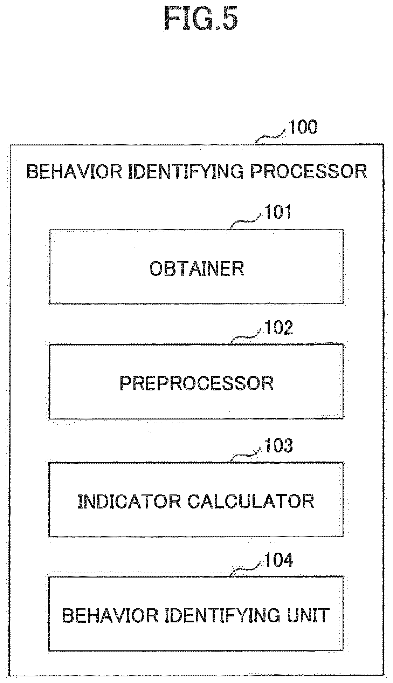

Next, a functional configuration of the behavior identifying processor 100 according to the present embodiment will be described with reference to FIG. 5. FIG. 5 is a diagram illustrating an example of a functional configuration of the behavior identifying processor 100 according to the first embodiment.

As illustrated in FIG. 5, the behavior identifying processor 100 includes an obtainer 101, a preprocessor 102, an indicator calculator 103, and a behavior identifying unit 104.

The obtainer 101 obtains measured data from the measured data memory unit 200. At this time, the obtainer 101 obtains measured data for a predetermined period of time (e.g., 10 minutes) from the measured data memory unit 200, for example, for each tag ID.

The preprocessor 102 applies preprocessing to the measured data obtained by the obtainer 101. The preprocessing includes, for example, missing data completion (resampling) of measured data, noise removal, and the like. The preprocessor 102 calculates the L2 norms of acceleration sensor values included in the measured data after the missing data completion, and performs noise removal by using the calculated L2 norms.

The indicator calculator 103 calculates indicators from the L2 norms of the acceleration sensor values included in the measured data after the preprocessing by the preprocessor 102. For example, the indicator calculator 103 calculates, as indicators, the maximum value and the standard deviation from the L2 norms of the acceleration sensor values included in the measured data after the preprocessing.

The behavior identifying unit 104 identifies one of the four behaviors of "standing", "lying", "ruminating", and "moving" from the maximum value and the standard deviation calculated by the indicator calculator 103 and the behavior identifying model 300. Also, in the case of having identified "moving" as the behavior, the behavior identifying unit 104 further identifies one of detailed behaviors ("feeding", "walking", and "water-drinking") from RSSI values included in the measured data. Note that the behavior identifying unit 104 may identify one of the four behaviors from the maximum value, the standard deviation, and a processed result by a program or the like that generates data equivalent to the behavior identifying model 300.

Note that information representing a behavior identified by the behavior identifying unit 104 may be stored in, for example, a DB (database) or a file implemented by an auxiliary storage device such as an HDD or SSD, or may be displayed on a display device such as a display. Also, the information may be output to another device (e.g., a PC, smartphone, tablet terminal, or the like) connected to the behavior identifying device 10.

### <Behavior identification process>

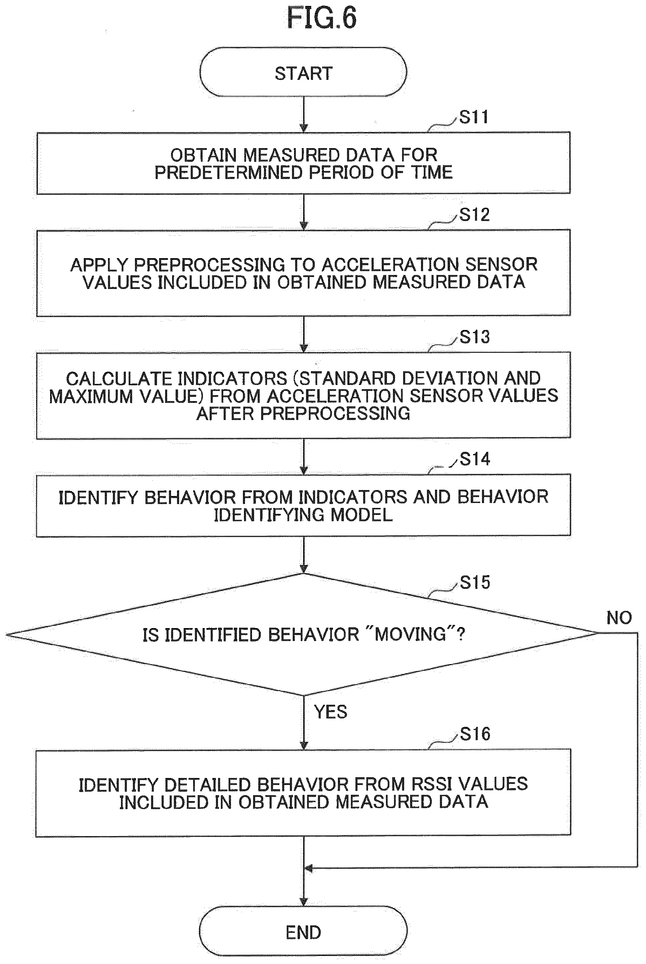

Next, a behavior identifying process according to the present embodiment will be described with reference to FIG. 6. FIG. 6 is a flow chart illustrating an example of a behavior identifying process according to the first embodiment. Note that the behavior identifying process illustrated in FIG. 6 may be executed, for example, at a date and time set in advance, or may be repeatedly executed every predetermined interval (e.g., 24 hours).

First, the obtainer 101 obtains measured data for a predetermined period of time (e.g., 10 minutes) from the measured data memory unit 200 for each tag ID (Step S11). In this way, the obtainer 101 obtains measured data in a predetermined unit of time (e.g., unit of 10 minutes) from the measured data memory unit 200 for each cow (tag ID). Note that such a predetermined unit of time is not limited to 10 minutes, and can be set, for example, by the user of the behavior identifying device 10 with any length of time.

In the following, the description will be continued for a case where measured data 1, measured data 2, ..., and measured data N₁ of a tag having an ID "Tag1" for 10 minutes are obtained by the obtainer 101.

Next, the preprocessor 102 applies preprocessing to the measured data obtained by the obtainer 101 (Step S12). In other words, the preprocessor 102 performs missing data completion (resampling), noise removal, and the like with respect to the measured data. Note that missing data completion is a process of complementing missing data when data cannot be obtained, in order to improve the accuracy of collected data. Also, noise removal is a process of removing data that represents an instantaneous movement of a cow (e.g., a movement of momentarily shaking the body or a movement of trembling). As a result, measured data 1, measured data 2, ..., measured data N₂ after the preprocessing are obtained.

Next, the indicator calculator 103 calculates indicators (the maximum value and the standard deviation of the L2 norms) from the L2 norms of acceleration sensor values included in each item of the measured data after the preprocessing by the preprocessor 102 (Step S13). In other words, denoting the L2 norms of the acceleration sensor values included in the measured data after the preprocessing by a1, a2, ..., aN₂, the standard deviation σ and the maximum value m are calculated for these a1, a2, ..., aN₂. Note that the maximum value m is a maximum value from among differences between the average value of the L2 norms a1, a2, ..., aN₂ and the respective L2 norms ai (i = 1, ..., N₂).

Next, the behavior identifying unit 104 identifies one of the four behaviors of "standing", "lying", "ruminating", and "moving" from the standard deviation σ and the maximum value m calculated by the indicator calculator 103 and the behavior identifying model 300 (Step S14). In other words, the behavior identifying unit 104 identifies the behavior by identifying which of the regions D1 to D4 on the behavior identifying model 300 includes the standard deviation σ and the maximum value m calculated by the indicator calculator 103.

Next, the behavior identifying unit 104 determines whether or not the behavior identified at Step S14 is "moving" (Step S15).

If having determined at Step S15 that the identified behavior is not "moving" (i.e., if the identified behavior is "standing", "lying", or "ruminating"), the behavior identifying processor 100 ends the process.

On the other hand, if having determined at Step S15 that the identified behavior is "moving", the behavior identifying unit 104 identifies a detailed behavior ("feeding", "walking", or "water-drinking") from the RSSI values included in the measured data 1, measured data 2, ..., measured data N2 after the preprocessing at Step S12 (Step S16).

In other words, for example, among the RSSI values included in the measured data 1, measured data 2, ..., measured data N2, if an RSSI value of a radio wave received from the leaky coaxial antenna 301 or the leaky coaxial antenna 302 exceeds a predetermined threshold value, the behavior identifying unit 104 identifies the detailed behavior as "feeding" or "water-drinking". On the other hand, if neither of the RSSI value of the radio wave received from the leaky coaxial antenna 301 nor the RSSI value of the radio wave received from the leaky coaxial antenna 302 exceeds the predetermined threshold value, the behavior identifying unit 104 identifies the detailed behavior as "walking".

As described above, the behavior identifying system 1 according to the present embodiment can identify a behavior of a cow from measured data received from the tag 20 attached to the cow. At this time, the behavior identifying system 1 according to the present embodiment identifies the behavior of the cow by using indicators calculated from acceleration sensor values included in the measured data and the behavior identifying model 300 generated in advance by a machine learning method. In particular, by using the standard deviation and the maximum value of the L2 norms as the indicators, it is possible to identify "ruminating" as a behavior peculiar to cows with high accuracy. In other words, it is possible to distinguish "ruminating" as a behavior peculiar to cows from "standing", "lying", and the like with high accuracy.

By displaying behaviors of cows identified in this way, for example, on a display device (a display or the like) of the behavior identifying device 10 to be presented to a dairy farmer or the like, it is possible to easily confirm behaviors often observed during the estrus. Also, this also leads to early detection of abnormal behaviors due to illness or injury, and makes health care for the cows easier.

### [Second embodiment]

Next, a second embodiment will be described. In the first embodiment, one of the four behaviors of a cow ("standing", "lying", "ruminating", and "moving") are to be identified by using acceleration sensor values; however, among these behaviors, in some cases, it is difficult to distinguish "standing" and "lying" from each other. In other words, for example, there is a case where a cow is "standing" while moving the body minutely, and there is also a case where the cow is "lying" while moving the body considerably. In such cases, it may be difficult for the motion-activeness analysis described above to distinguish "standing" and "lying" from each other (i.e., "standing" or "lying" may be identified erroneously).

Similarly, for example, considering that a cow is "ruminating", there is a case where a cow is ruminating in a standing state, and there is also a case where the cow is ruminating in a lying state. In such cases, it may be difficult for the motion-activeness analysis described above to distinguish "standing" and "lying" from each other.

Thereupon, in the second embodiment, a case will be described in which "standing" or "lying" is identified with higher accuracy by using an atmospheric pressure sensor.

Note that in the second embodiment, different points from the first embodiment will be mainly described, and the description may be omitted appropriately for elements having substantially the same functions as in the first embodiment and elements performing substantially the same processing.

### <Overall configuration>

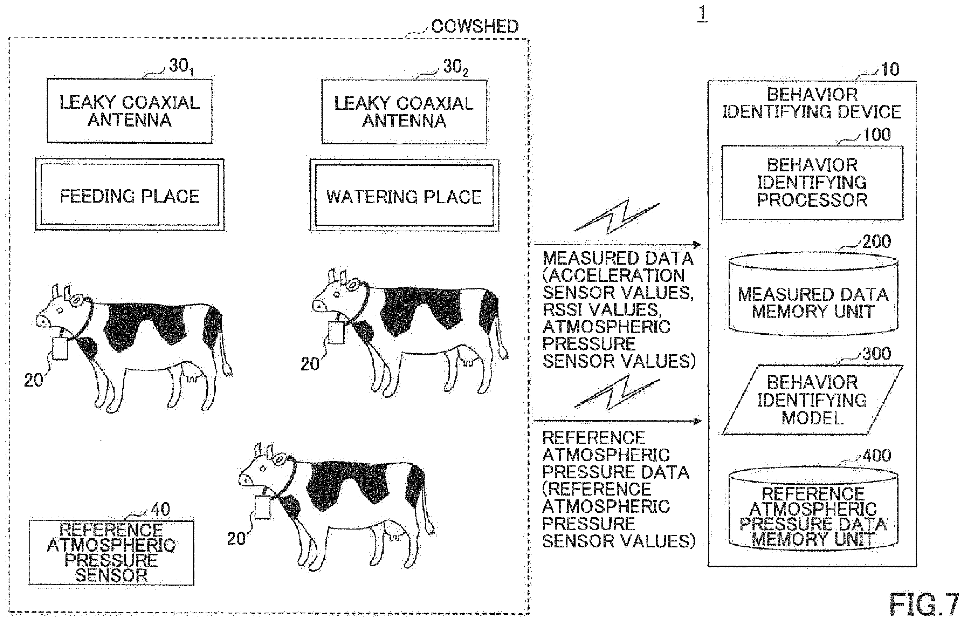

First, an overall configuration of a behavior identifying system 1 according to the present embodiment will be described with reference to FIG. 7. FIG. 7 is a diagram illustrating an example of an overall configuration of the behavior identifying system 1 according to the second embodiment.

As illustrated in FIG. 7, the behavior identifying system 1 according to the present embodiment includes a reference atmospheric pressure sensor 40 to measure reference atmospheric pressure.

The reference atmospheric pressure sensor 40 is installed at a predetermined position in a cowshed (e.g., above the ground in the cowshed) to measure reference atmospheric pressure. The reference atmospheric pressure sensor 40 transmits reference atmospheric pressure data including a reference atmospheric pressure sensor value that represents a measured atmospheric pressure to the behavior identifying device 10 every predetermined interval (e.g., every two seconds). The reference atmospheric pressure data transmitted to the behavior identifying device 10 is accumulated (stored) in a reference atmospheric pressure data memory unit 400, which will be described later.

Also, a tag 20 according to the present embodiment further includes an atmospheric pressure sensor to measure atmospheric pressure. Further, the tag 20 transmits measured data including an atmospheric pressure sensor value to the behavior identifying device 10 every predetermined interval (e.g., every two seconds). Note that the tag 20 does not necessarily need to include an atmospheric pressure sensor; for example, an atmospheric pressure sensor may be attached to the cow separately from the tag 20.

The behavior identifying device 10 according to the present embodiment further includes the reference atmospheric pressure data memory unit 400. The reference atmospheric pressure data memory unit 400 stores reference atmospheric pressure data received from the reference atmospheric pressure sensor 40. The reference atmospheric pressure data memory unit 400 may be implemented by using an auxiliary storage device such as an HDD or SSD. The reference atmospheric pressure data memory unit 400 stores multiple reference atmospheric pressure data items for predetermined intervals (e.g., every two seconds).

The behavior identifying processor 100 according to the present embodiment further identifies a behavior of a cow (among "standing" and "lying") based on atmospheric pressure sensor values included in the measured data stored in the measured data memory unit 200 and reference atmospheric pressure sensor values included in the reference atmospheric pressure data stored in the reference atmospheric pressure data memory unit 400.

### <Identification of "standing" and "lying" by atmospheric pressure sensor>

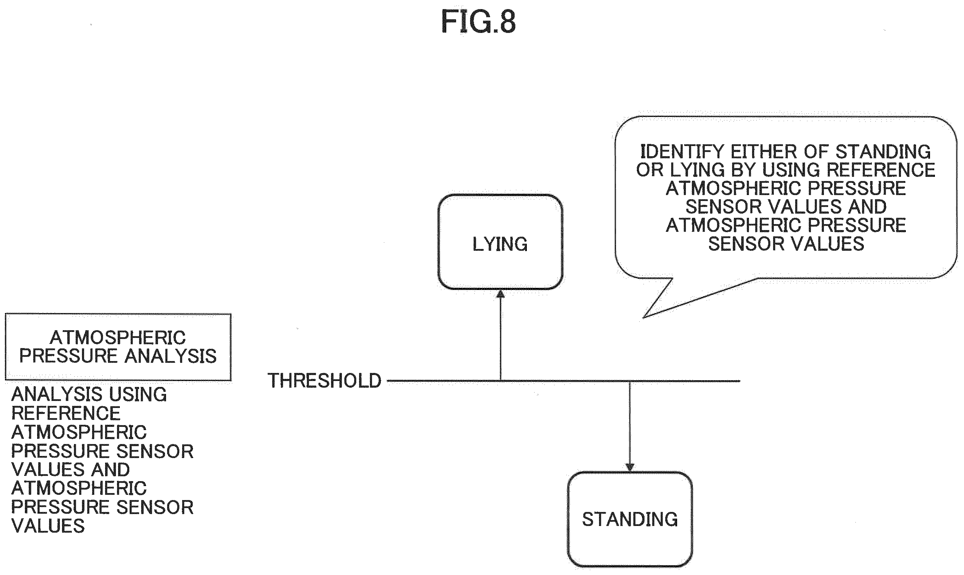

Here, an outline of a method of identifying "standing" and "lying" by an atmospheric pressure sensor among the behaviors of a cow to be identified by the behavior identifying system according to the present embodiment will be described with reference to FIG. 8. FIG. 8 is a diagram illustrating an example of a case of identifying "standing" or "lying" by atmospheric pressure analysis.

Which behavior among "standing" and "lying" a cow is performing is identified by the atmospheric pressure analysis in addition to the motion-activeness analysis described in the first embodiment. The atmospheric pressure analysis according to the present embodiment identifies a behavior of a cow as either of "standing" or "lying" based on atmospheric pressure sensor values included in the measured data and reference atmospheric pressure sensor values included in the reference atmospheric pressure data. More specifically, the atmospheric pressure analysis according to the present embodiment calculates differences between atmospheric pressure sensor values and reference sensor values, respectively, for a predetermined period of time (e.g., 24 hours). Then, if a calculated difference (this difference is referred to as an "atmospheric pressure sensor difference value") exceeds a predetermined threshold value, the behavior is identified as "lying". On the other hand, if the atmospheric pressure sensor difference value does not exceed the predetermined threshold value, the behavior is identified as "standing". Using atmospheric pressure sensor difference values measured by the reference atmospheric pressure sensor 40 enables to determine, for example, whether a measured value of the atmospheric pressure sensor included in the tag 20 attached to a cow has increased or decreased relatively, regardless of the weather (approaching low atmospheric depression, etc.).

In this way, by using atmospheric pressure sensor values measured by the atmospheric pressure sensor to identify the behavior of a cow as "lying" if the atmospheric pressure sensor difference value exceeds the threshold value, or as "standing" if not exceeding the threshold value, "standing" and "lying" among behaviors of the cow can be identified with high accuracy.

### <Measured data stored in measured data memory unit 200>

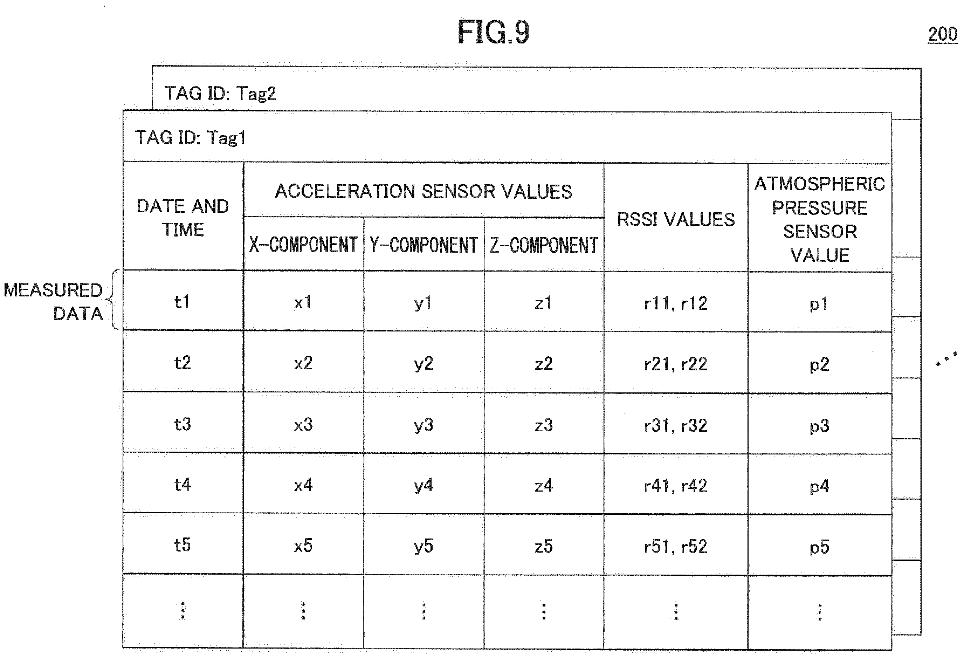

Here, measured data stored in the measured data memory unit 200 according to the present embodiment will be described with reference to FIG. 9. FIG. 9 is a diagram illustrating an example of measured data stored in the measured data memory unit 200 according to the second embodiment. Note that in the case of receiving measured data from the tag 20, the behavior identifying device 10 may cause the behavior identifying processor 100 to store (accumulate) the received measured data in the measured data memory unit 200.

As illustrated in FIG. 9, the measured data memory unit 200 stores one or more items of measured data for each tag ID that identifies a tag. The measured data according to the present embodiment further includes an atmospheric pressure sensor value. An atmospheric pressure sensor value is a value of atmospheric pressure measured by the atmospheric pressure sensor included in the tag 20.

In this way, in the measured data stored in the measured data memory unit 200 according to the present embodiment, measured data including a date and time, acceleration sensor values, RSSI values, and an atmospheric pressure sensor value is accumulated (stored) for each tag ID.

### <Functional Configuration of behavior identifying processor 100>

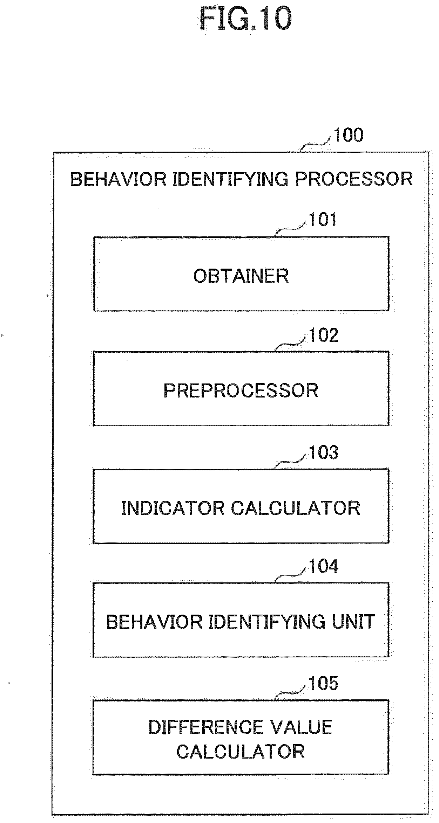

Next, a functional configuration of the behavior identifying processor 100 according to the present embodiment will be described with reference to FIG. 10. FIG. 10 is a diagram illustrating an example of a functional configuration of the behavior identifying processor 100 according to the second embodiment.

As illustrated in FIG. 10, the behavior identifying processor 100 according to the present embodiment further includes a difference value calculator 105. Also, an obtainer 101 and a behavior identifying unit 104 according to the present embodiment have different functions from those in the first embodiment.

When performing behavior identification by position analysis, the obtainer 101 according to the present embodiment obtains measured data for a predetermined period of time (e.g., 24 hours) from the measured data memory unit 200 for each tag ID, and obtains reference atmospheric pressure data for the predetermined period of time from the reference atmospheric pressure data memory unit 400.

The difference value calculator 105 calculates atmospheric pressure sensor difference values each representing a difference between an atmospheric pressure sensor value included in the measured data obtained by the obtainer 101 and a reference atmospheric pressure sensor value included in the reference atmospheric pressure data.

Note that the preprocessor 102 may apply preprocessing to the measured data obtained by the obtainer 101. In this case, the preprocessor 102 may perform missing data completion, noise removal (i.e., noise removal in the atmospheric pressure sensor values), and the like with respect to the measured data. Then, in this case, the difference value calculator 105 may calculate atmospheric pressure sensor difference values from the atmospheric pressure sensor values included in the measured data preprocessed by the preprocessor 102 and the reference atmospheric pressure sensor values included in the reference atmospheric pressure data.

The behavior identifying unit 104 determines whether or not an atmospheric pressure sensor difference value calculated by the differential value calculator 105 exceeds a predetermined threshold value, to identify whether the behavior of a cow is "standing" or "lying". Note that as the predetermined threshold value, for example, an intermediate value between the maximum value and the minimum value of the atmospheric pressure sensor difference values calculated from the atmospheric pressure sensor values included in the measured data obtained by the obtainer 101, may be used. This is because a cow is considered to perform "standing" and "lying", for example, at least once in 24 hours.

Note that, for example, in the case where the tag 20 is attached to a collar or the like of a cow, an atmospheric pressure sensor value may be increased by a behavior of "feeding" or "water-drinking" by the cow. This is because a cow performs feeding or drinking water by lowering the neck in a standing position. In the case where the tag 20 is attached to a collar or the like of a cow, it is often the case that the height of the tag 20 from the ground when a cow performs "feeding" or "water-drinking" is roughly the same as the height of the tag 20 from the ground when a cow is "lying". Therefore, the obtainer 101 may obtain measured data including hours before and after a behavior of "feeding" or "water-drinking" was identified by the motion-activeness analysis and position analysis, to set an intermediate value between the maximum value and the minimum value of the atmospheric pressure sensor difference values calculated from the atmospheric pressure sensor values included in the obtained measured data, as the threshold value.

### <Identification process for standing or lying behavior>

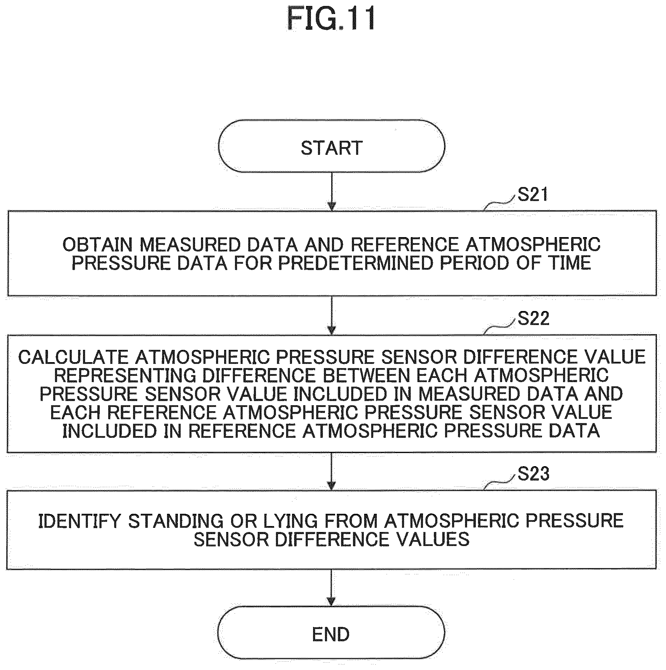

Next, a behavior identifying process according to the present embodiment will be described with reference to FIG. 11. FIG. 11 is a flow chart illustrating an example of the behavior identifying process for standing or lying behavior according to the second embodiment. Note that the behavior identifying process illustrated in FIG. 11 may be executed, for example, at a date and time set in advance, or may be repeatedly executed every predetermined interval (e.g., 24 hours).

First, the obtainer 101 obtains, for each tag ID, measured data for a predetermined period of time (e.g., 24 hours) from the measured data memory unit 200, and obtains reference atmospheric pressure data for the predetermined period of time from the reference atmospheric pressure data memory unit 400 (Step S21).

In the following, the description will be continued for the case where measured data 1, measured data 2, ..., and measured data N₄ for 24 hours of a tag having an ID "Tag1", and reference atmospheric pressure data 1, reference atmospheric pressure data 2, ..., and reference atmospheric pressure data N₄ for the 24 hours are obtained by the obtainer 101. Note that for the sake of simplicity, it is assumed in the description that the measured data i and the reference atmospheric pressure data i (i = 1, 2, ..., N₄) are data at the same date and time, respectively.

Next, the difference value calculator 105 calculates atmospheric pressure sensor difference values each representing a difference between an atmospheric pressure sensor value included in the measured data obtained by the obtainer 101 and a reference atmospheric pressure sensor value included in the reference atmospheric pressure data (Step S22) .

In other words, the difference value calculator 105 calculates an atmospheric pressure sensor difference value 1 between an atmospheric pressure sensor value included in the measured data 1 and a reference atmospheric pressure sensor value included in the reference atmospheric pressure data 1. The difference value calculator 105 also calculates an atmospheric pressure sensor difference value 2 between an atmospheric pressure sensor value included in the measured data 2 and a reference atmospheric pressure sensor value included in the reference atmospheric pressure data 2. Similarly, the difference value calculator 105 calculates an atmospheric pressure sensor difference value N₄ between an atmospheric pressure sensor value included in the measured data N₄ and a reference atmospheric pressure sensor value included in the reference atmospheric pressure data N₄.

Next, the behavior identifying unit 104 determines whether or not an atmospheric pressure sensor difference value calculated by the differential value calculator 105 exceeds a predetermined threshold value, to identify whether the behavior of the cow is "standing" or "lying" (Step S23). In other words, the behavior identifying unit 104 determines whether the atmospheric pressure sensor difference value 1, the atmospheric pressure sensor difference value 2, ..., or the atmospheric pressure sensor difference value N₄ exceeds the predetermined threshold value, to identify whether the behavior of the cow is "standing" or "lying".

For example, if a certain atmospheric pressure sensor difference value exceeds the predetermined threshold value, the behavior identifying unit 104 identifies the behavior of the cow as "lying" at the date and time for which the atmospheric pressure sensor difference value is calculated. On the other hand, if a certain atmospheric pressure sensor difference value does not exceed a predetermined threshold value, the behavior identifying unit 104 identifies the behavior of the cow as "standing" at the date and time for which the atmospheric pressure sensor difference value is calculated.

The behavior identifying unit 104 may take into account a result of the motion-activeness analysis to consider whether the behavior of the cow is "standing" or "lying". More specifically, at a date and time at which the behavior of the cow is identified as "ruminating" or "moving" by the motion-activeness analysis, the atmospheric pressure analysis may not be performed. Meanwhile, if the behavior of the cow is identified as "standing" or "lying" by the motion-activeness analysis, measured data and reference atmospheric pressure data within a predetermined range including the identified date and time may be obtained to perform the atmospheric pressure analysis.

In other words, if the behavior of the cow is identified as "ruminating" or "moving" by the motion-activeness analysis, the atmospheric pressure analysis may be not performed; on the other hand, if the behavior of the cow is identified as "standing" or "lying" by the motion-activeness analysis, the atmospheric pressure analysis may be performed. Thereby, if "standing" or "lying" is identified by the motion-activeness analysis, the atmospheric pressure analysis is performed for identifying "standing" or "lying" with higher accuracy. In this way, the atmospheric pressure analysis may be performed depending on a result of the motion-activeness analysis.

As described above, the behavior identifying system 1 according to the present embodiment can identify a "standing" or "lying" behavior of a cow by the atmospheric pressure analysis using an atmospheric pressure sensor. Moreover, the behavior identifying system 1 according to the present embodiment can identify one of the two behaviors of "standing" and "lying" with high accuracy as compared to the case of using the motion-activeness analysis singly, by using the atmospheric pressure analysis singly or in combination with the motion-activeness analysis.

### [Third embodiment]

Next, a third embodiment will be described. In the third embodiment, a case will be described in which the cowshed is a free-barn type.

In general, free-stall type cowsheds and free-barn type cowsheds have been known as cowsheds. Unlike free-stall type cowsheds, it is often the case that there are mounds in free-barn type cowsheds. For this reason, in a free-barn type cowshed, behavioral identification by the atmospheric pressure analysis may be incorrect.

For example, suppose that an atmospheric pressure sensor value in the case of a cow "standing" on the flat ground at the foot of a mound is roughly the same as an atmospheric pressure sensor value in the case of a cow "lying" on the mound. In this case, "lying" on the mound may be erroneously identified as "standing".

Thereupon, in the third embodiment, a case will be described in which "standing" and "lying" can be identified with high accuracy even in a free-barn type cowshed by elaborating a range in which measured data and reference atmospheric pressure data are obtained.

Note that in the third embodiment, different points from the second embodiment will be mainly described, and the description may be omitted appropriately for elements having substantially the same functions as in the second embodiment and elements performing substantially the same processing.

### <Obtainment of measured data in free-barn type cowshed>

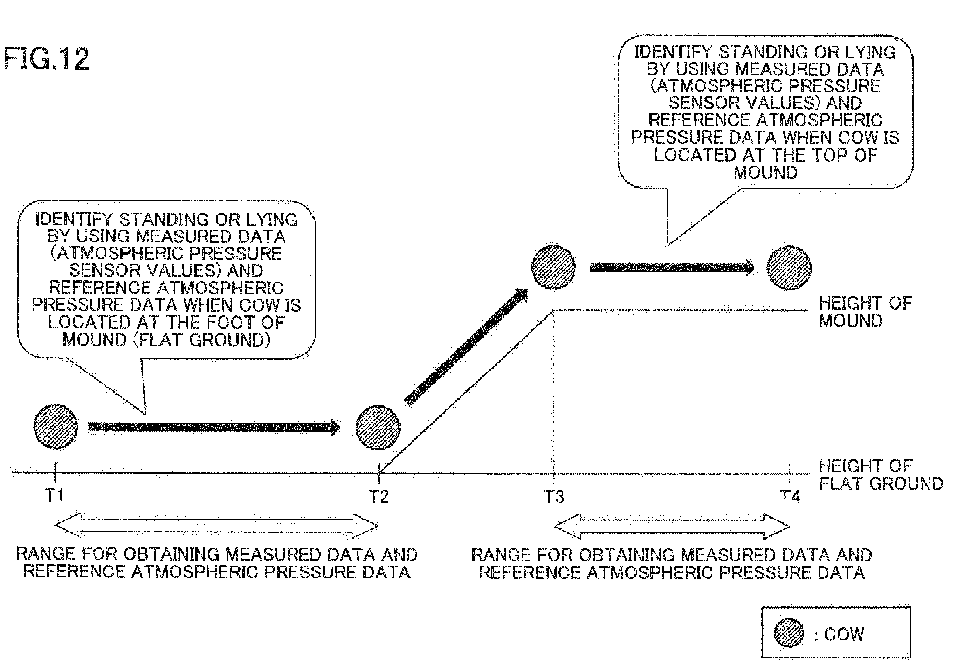

In the case of the cowshed being a free-barn type, the range of measured data reference atmospheric pressure data obtained by the obtainer 101 is elaborated at Step S21 in FIG. 11. This will be described with reference to FIG. 12. FIG. 12 is a diagram illustrating an example of a range in which measured data and reference atmospheric pressure data are obtained in a free-barn type cowshed.

As illustrated in FIG. 12, assume that a cow is on the flat ground (at the foot of a mound) during time T1 to T2, climbs up the mound during time T2 to T3, and is on the mound during time T3 to T4. In this case, in order to identify the behavior of the cow on the flat ground, atmospheric pressure analysis is performed using measured data and reference atmospheric pressure data during the time T1 to T2 during which the cow is on the flat ground. In contrast, in order to identify the behavior of the cow on the mound, atmospheric pressure analysis is performed using measured data and reference atmospheric pressure data during the time T3 to T4 during which the cow is on the mound.

Whether the cow is on the flat ground, climbing up the mound, or on the mound can be determined by using acceleration sensor values and atmospheric pressure sensor values. In other words, for example, if the atmospheric pressure sensor value hardly changes for a certain period of time while the acceleration sensor value changes, it is possible to determine that the cow is on the flat ground. Also, for example, if both the atmospheric pressure sensor value and the acceleration sensor values change for a certain period of time, it is possible to determine that the cow is climbing up the mound. Further, for example, after the cow has been determined as climbing up the mound, if the cow is determined as being on the flat ground, it is possible to determine that the cow is on the mound. Note that it is possible to determine that the cow is climbing down the mound similarly to the case of climbing up the mound.

Note that a cow may be "lying" in the course of climbing up the mound (or climbing down the mound). Also, a cow may be "lying", and then, "standing" up in the course of climbing up the mound (or climbing down the mound). These cases may be determined, for example, from changing patterns of the acceleration sensor value or changing patterns of the atmospheric pressure sensor value in the course of climbing up the mound (or climbing down the mound).

As described above, the behavior identifying system 1 according to the present embodiment can identify a "standing" or "lying" behavior of a cow by the atmospheric pressure analysis using an atmospheric pressure sensor even in a free-barn type cowshed.

### [Fourth embodiment]

Next, a fourth embodiment will be described. In the first to third embodiments, cases have been described in which the behavior of a cow is identified by using the leaky coaxial antenna 30. However, there may be a case where it is difficult to install a leaky coaxial antenna 30 in a cowshed or the like.

Thereupon, in the fourth embodiment, a case will be described in which the behavior of a cow is identified without using a leaky coaxial antenna 30.

Note that in the fourth embodiment, different points from the second embodiment will be mainly described, and the description may be omitted appropriately for elements having substantially the same functions as in the second embodiment and elements performing substantially the same processing.

### <Overall configuration>

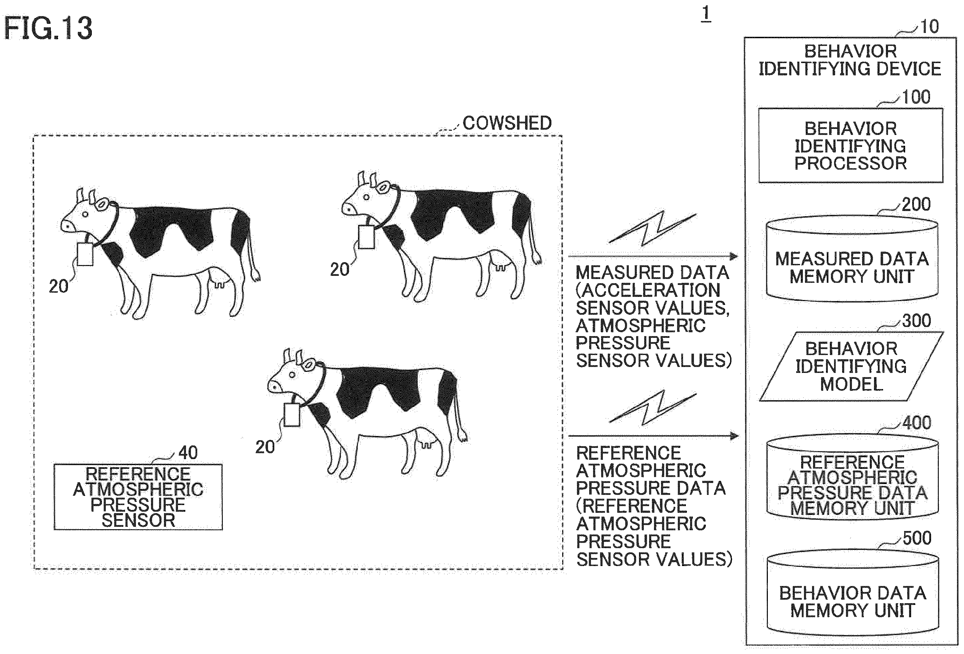

First, an overall configuration of a behavior identifying system 1 according to the present embodiment will be described with reference to FIG. 13. FIG. 13 is a diagram illustrating an example of an overall configuration of the behavior identifying system 1 according to the fourth embodiment.

As illustrated in FIG. 1, the behavior identifying system 1 according to the present embodiment does not include a leaky coaxial antenna 30. Also, a tag 20 according to the present embodiment does not include a radio wave sensor that measures received signal strength of a radio wave received from a leaky coaxial antenna 30.

The behavior identifying device 10 according to the present embodiment includes a behavior data memory unit 500. Also, the behavior identifying processor 100 according to the present embodiment identifies a behavior of a cow by motion-activeness analysis based on measured data stored in a measured data memory unit 200 and a behavior identifying model 300. Then, once a predetermined behavior has been identified by the motion-activeness analysis, the behavior identifying processor 100 identifies the behavior of the cow more precisely by atmospheric pressure analysis based on the measured data stored in the measured data memory unit 200, the reference atmospheric pressure data stored in the reference atmospheric pressure data memory unit 400, and the behavior data stored in the behavior data memory unit 500.

The behavior data memory unit 500 stores behavior data that represents behaviors identified by the behavior identifying processor 100. The behavior data memory unit 500 can be implemented by an auxiliary storage device such as an HDD or SSD. The behavior data memory unit 500 stores behavior data that represents behaviors identified by the behavior identifying processor 100 at predetermined time intervals (e.g., every 10 minutes).

### <Identification of behavior of cow>

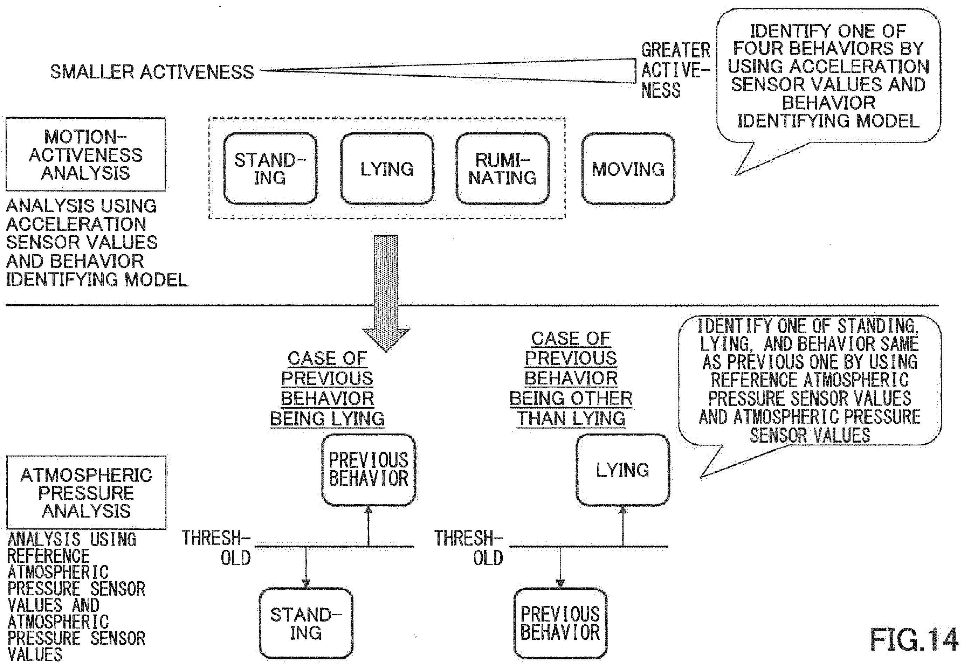

Here, behaviors of a cow to be identified by the behavior identifying system 1 according to the present embodiment, and an outline of an identification method will be described with reference to FIG. 14. FIG. 14 is a diagram illustrating an example of behavior identification of a cow.

First, assume that behaviors of a cow to be identified by the behavior identifying system 1 according to the present embodiment include four behaviors of "standing", "lying", "ruminating", and "moving" in ascending order of activeness of the motions. Therefore, it is assumed in the present embodiment that one of the four behaviors of "standing", "lying", "ruminating", and "moving" performed by a cow is identified.

Which one of the behaviors of "standing", "lying", "ruminating", and "moving" the cow is performing is identified by the motion-activeness analysis as in the second embodiment.

Also, in the present embodiment, if the behavior is identified as "standing", "lying", or "ruminating" by the motion-activeness analysis, the behavior is identified more precisely by the atmospheric pressure analysis. This is because in some cases, the motion-activeness analysis cannot precisely identify the posture of the cow performing the behavior identified by using the behavior identifying model 300.

The atmospheric pressure analysis according to the present embodiment identifies a behavior of a cow, based on a minimum atmospheric pressure difference value that represents a minimum value from among differences between atmospheric pressure sensor values included in the measured data and reference atmospheric pressure sensor values included in the reference atmospheric pressure data, respectively, for a predetermined period of time (e.g., five minutes), and a predetermined threshold value.

More specifically, for example, if the behavior 10 minutes ago (i.e., the previous behavior) is "lying", the atmospheric pressure analysis according to the present embodiment determines whether or not a first atmospheric pressure difference minimum value between five minutes ago and the present time has decreased from a second atmospheric pressure difference minimum value between 10 minutes ago and five minutes ago by a predetermined threshold value or greater. Then, if the first minimum atmospheric pressure difference value has decreased from the second minimum atmospheric pressure difference value by the predetermined threshold value or greater, the behavior is identified as "standing". On the other hand, if the first minimum atmospheric pressure difference value has not decreased from the second minimum atmospheric pressure difference value by the predetermined threshold value or greater, the behavior is identified as the same behavior as the previous one (i.e., "lying").

Also, for example, if the behavior 10 minutes ago (i.e., the previous behavior) is other than "lying" (i.e., "standing" or "ruminating"), the atmospheric pressure analysis determines whether or not the first atmospheric pressure difference minimum value has increased from the second atmospheric pressure difference minimum value by a predetermined threshold value or greater. Then, if the first minimum atmospheric pressure difference value has increased from the second minimum atmospheric pressure difference value by the predetermined threshold value or greater, the behavior is identified as "lying". On the other hand, if the first minimum atmospheric pressure difference value has not increased from the second minimum atmospheric pressure difference value by the predetermined threshold value or greater, the behavior is identified as the same behavior as the previous one (i.e., "standing" or "ruminating").

As above, the behavior identifying system 1 according to the present embodiment identifies one of the four behaviors of "standing", "lying", "ruminating", and "moving" from acceleration sensor values received from the tag 20 attached to a cow. Further, if the behavior of the cow is identified as "standing", "lying" or "ruminating", the behavior identifying system 1 according to the present embodiment identifies the behavior more precisely from atmospheric pressure sensor values and reference atmospheric pressure sensor values.

Thereby, it is possible to identify the behavior of the cow as one of "standing", "lying", "ruminating", and "moving". Also, at this time, the atmospheric pressure analysis enables to identify "standing", "lying", or "ruminating" with high accuracy. Further, according to the atmospheric pressure analysis according to the present embodiment, "standing", "lying", or "ruminating" can be identified with high accuracy even in a cowshed where there is a mound or the like, such as a free-barn type cowshed.

Therefore, by displaying behaviors of cows identified in this way, for example, on a display device (a display or the like) of the behavior identifying device 10, it is possible to easily confirm behaviors often observed during the estrus. Also, this also leads to early detection of abnormal behaviors due to illness or injury, and makes health care for the cows easier.

### <Measured data stored in measured data memory unit 200>

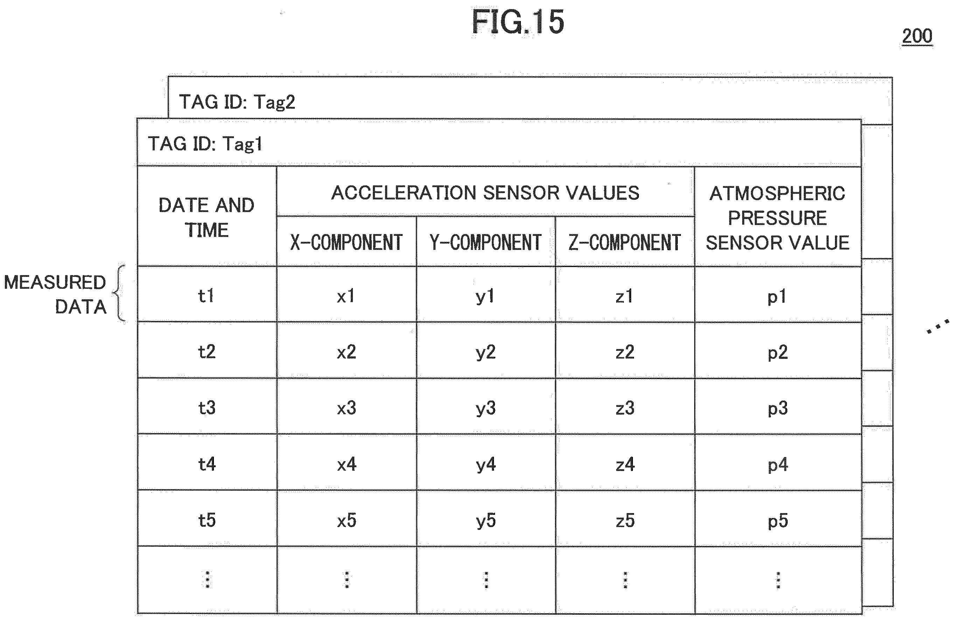

Here, measured data stored in the measured data memory unit 200 according to the present embodiment will be described with reference to FIG. 15. FIG. 15 is a diagram illustrating an example of measured data stored in the measured data memory unit 200 according to the present embodiment.

As illustrated in FIG. 15, the measured data memory unit 200 stores one or more items of measured data for each tag ID that identifies a tag. Measured data according to the present embodiment includes date and time, acceleration sensor values, and an atmospheric pressure sensor value. In other words, the measured data according to the present embodiment does not include RSSI values.

In this way, in the measured data stored in the measured data memory unit 200 according to the present embodiment, measured data including date and time, acceleration sensor values, and an atmospheric pressure sensor value are accumulated (stored) for each tag ID.

### <Functional Configuration of behavior identifying processor 100>

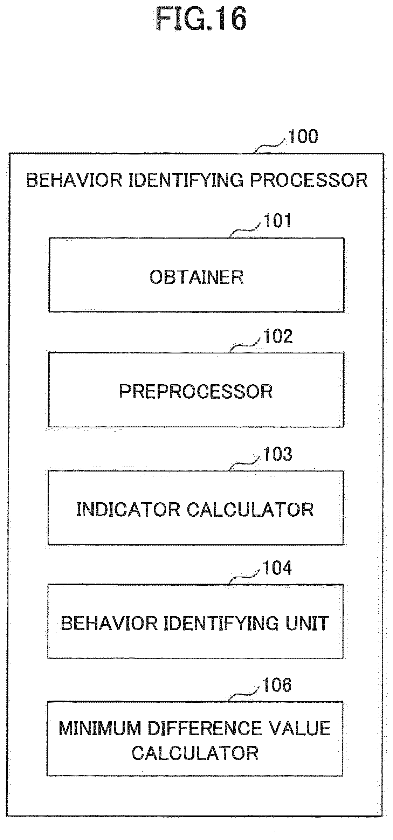

Next, a functional configuration of the behavior identifying processor 100 according to the present embodiment will be described with reference to FIG. 16. FIG. 16 is a diagram illustrating an example of a functional configuration of the behavior identifying processor 100 according to the fourth embodiment.

As illustrated in FIG. 16, the behavior identifying processor 100 according to the present embodiment includes a minimum difference value calculator 106 instead of a difference value calculator 105.

Also, an obtainer 101 according to the present embodiment obtains the latest behavior data (i.e., behavior data that represents the identified previous behavior) from among behavior data stored in the behavior data memory unit 500.

The minimum difference value calculator 106 calculates a minimum atmospheric pressure difference value from measured data and reference atmospheric pressure data obtained by the obtainer 101. In other words, the minimum difference value calculator 106 calculates, from the measured data and reference atmospheric pressure data obtained by the obtainer 101, for example, for 10 minutes, a first minimum atmospheric pressure difference value for the latter-half five minutes and a second minimum atmospheric pressure difference value for the first-half five minutes.

A minimum atmospheric pressure difference value is a minimum value from among differences between atmospheric pressure sensor values included in the measured data for a predetermined period of time, and reference atmospheric pressure sensor values included in the reference atmospheric pressure data, respectively, for a predetermined period of time.

Also, in the case where an identified behavior is "standing", "lying" or "ruminating", the behavior identifying unit 104 according to the present embodiment identifies the behavior more precisely from the first minimum atmospheric pressure difference value and the second minimum atmospheric pressure difference value calculated by the minimum difference value calculator 106 (behavior identification by atmospheric pressure analysis), in accordance with the behavior represented by the behavior data obtained by the obtaining unit 101 (i.e., the identified previous behavior).

In other words, the behavior identifying unit 104 according to the present embodiment identifies the behavior as "standing" if the previous behavior is "lying", and the first minimum atmospheric pressure difference value has decreased from the second minimum atmospheric pressure difference value by a predetermined threshold value or greater. On the other hand, the behavior identifying unit 104 according to the present embodiment identifies the same behavior as previous one if the previous behavior is "lying", and the first minimum atmospheric pressure difference value has not decreased from the second minimum atmospheric pressure difference value by the predetermined threshold value or greater.

Furthermore, the behavior identifying unit 104 according to the present embodiment identifies the behavior as "lying" if the previous behavior is other than "lying", and the first minimum atmospheric pressure difference value has increased from the second minimum atmospheric pressure difference value by a predetermined threshold value or greater. On the other hand, the behavior identifying unit 104 according to the present embodiment identifies the same behavior as previous one if the previous behavior is other than "lying", and the first minimum atmospheric pressure difference value has not increased from the second minimum atmospheric pressure difference value by the predetermined threshold value or greater.

The predetermined threshold value is a value, for example, set in advance by the user. Such a threshold value is determined, for example, based on an empirical rule regarding the behavior identification of cows.

### <Behavior identification process>

Next, a behavior identifying process according to the present embodiment will be described.

### <<Behavior identification process by motion-activeness analysis>>

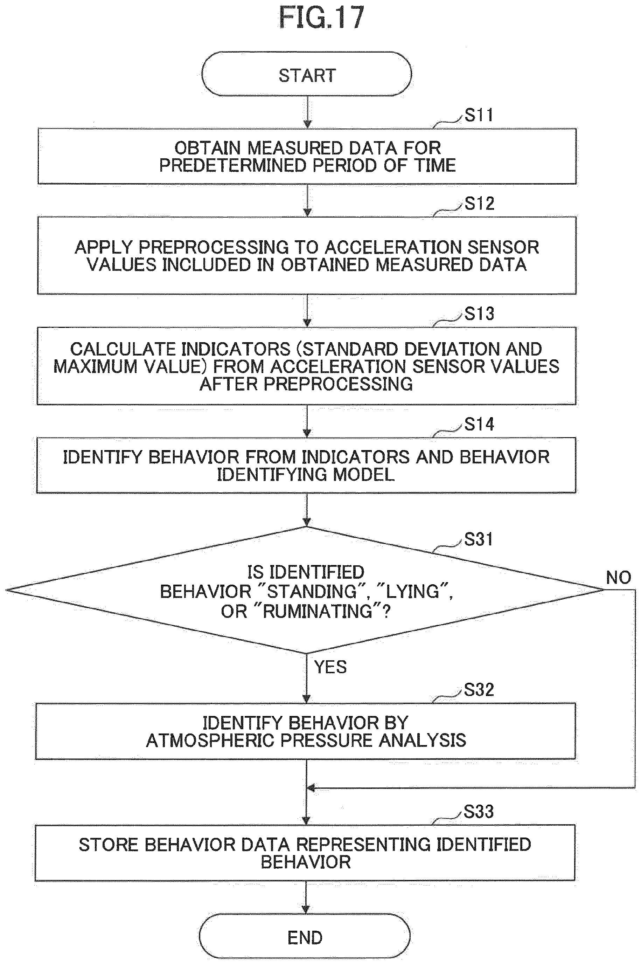

First, a behavior identifying process by motion-activeness analysis will be described with reference to FIG. 17. FIG. 17 is a flow chart illustrating an example of a behavior identifying process by motion-activeness analysis. Note that the behavior identifying process illustrated in FIG. 6 is repeatedly executed, for example, every 10 minutes. However, the behavior identifying process illustrated in FIG. 6 may be executed, for example, at date and time set in advance, or may be executed every predetermined interval.

Steps S11 to S14 in FIG. 17 are substantially the same as Steps S11 to S14 in FIG. 6, and the description is omitted here.

Next, the behavior identifying unit 104 determines whether the behavior identified at Step S14 is "standing", "lying", or "ruminating" (Step S31) .

If having determined at Step S31 that the identified behavior is "standing", "lying" or "ruminating", the behavior identifying processor 100 identifies the behavior more precisely by the atmospheric pressure analysis (Step S32). Details of the processing at this step will be described later.

On the other hand, if having determined at Step S31 that the identified behavior is not "standing", "lying" or "ruminating" (i.e., if the identified behavior is "moving"), or following Step S21, the behavior identifying unit 104 stores behavior data that represents the identified behavior in the behavior data memory unit 500 (Step S33). At this time, the behavior identifying unit 104 stores behavior data, for example, in which the identified behavior is associated with the current date and time, in the behavior data memory unit 500.

In other words, if having determined at Step S31 that the identified behavior is not "standing", "lying" or "ruminating", the behavior identifying unit 104 stores the behavior data in which the behavior of "moving" is associated with the current date and time in the behavior data memory unit 500. Similarly, following Step S32, the behavior identifying unit 104 stores the behavior data in which the behavior identified at Step S32 is associated with the current date and time, in the behavior data memory unit 500.

### <<Behavior identification process by atmospheric pressure analysis>>

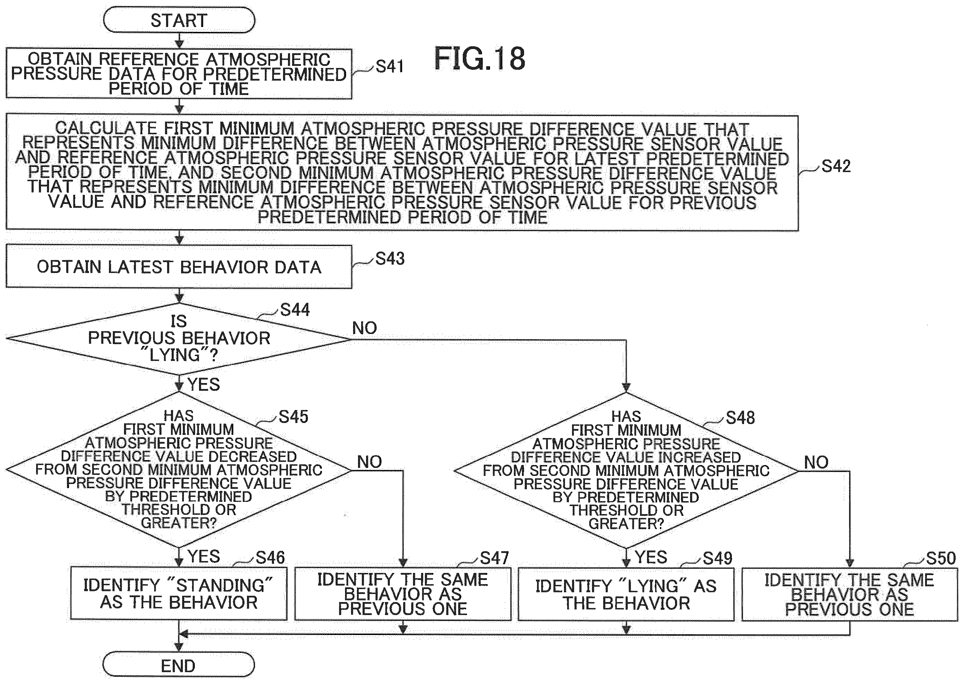

Here, details of the processing at Step S32 (a behavior identifying process by the atmospheric pressure analysis) will be described with reference to FIG. 18. FIG. 18 is a flow chart illustrating an example of a behavior identifying process based on the atmospheric pressure analysis.

Here, as described above, "ruminating" is divided into "ruminating while standing" and "ruminating while lying". Thereupon, FIG. 18 will be described assuming that "standing" includes "ruminating while standing" and "lying" includes "ruminating while lying".

First, the obtainer 101 obtains reference atmospheric pressure data for the same period of time as at Step S11 from the reference atmospheric pressure data memory unit 400 (Step S41). In other words, for example, in the case where the measured data for dates and times t1 to tN₃ (where N₃ is an integer greater than or equal to 1) has been obtained at Step S41, the obtainer 101 similarly obtains reference atmospheric pressure data for the dates and times t1 to tN₃ from the reference atmospheric pressure data memory unit 400.

Next, the minimum difference value calculator 106 calculates, from the measured data and the reference atmospheric pressure data obtained by the obtainer 101, a first minimum atmospheric pressure difference value for the latest predetermined period of time (e.g., the latest five minutes), and a second minimum atmospheric pressure difference value for a predetermined period of time before the latest (e.g., five minutes before the latest) (Step S42).

In other words, the minimum difference value calculator 106 calculates, for example, differences between atmospheric pressure sensor values and reference atmospheric pressure sensor values, respectively, for the latest five minutes, and takes a minimum difference from among the calculated differences as the first minimum atmospheric pressure difference value. Similarly, the minimum difference value calculator 106 calculates, for example, differences between atmospheric pressure sensor values and reference atmospheric pressure sensor values, respectively, for the previous five minutes (i.e., five minutes before the last five minutes), and takes a minimum difference from among the calculated differences as the second minimum atmospheric pressure difference value.

Note that the minimum difference value calculator 106 may use, for example, an average or a maximum value of differences between atmospheric pressure sensor values and reference atmospheric pressure sensor values, for a predetermined period of time. More specifically, for example, an "average atmospheric pressure difference value" that represents an average of differences between atmospheric pressure sensor values and reference atmospheric pressure sensor values for a predetermined period of time may be used instead of a "minimum atmospheric pressure difference value". Similarly, for example, a "maximum atmospheric pressure difference value" that represents a maximum value of differences between atmospheric pressure sensor values and reference atmospheric pressure sensor values for a predetermined period of time may be used instead of a "minimum atmospheric pressure difference value". However, in the case of using an "average atmospheric pressure difference value" or "maximum atmospheric pressure difference value", the accuracy of behavior identification is reduced compared to the case of using a "minimum atmospheric pressure difference value".

Next, the obtainer 101 obtains the latest behavior data from the behavior data stored in the behavior data memory unit 500 (i.e., behavior data that represents the identified previous behavior) (Step S43) .

Next, the behavior identifying unit 104 determines whether or not the behavior represented by the behavior data obtained at Step S43 is "lying" (Step S44). In other words, the behavior identifying unit 104 determines whether or not the identified previous behavior is "lying".

If having determined at Step S44 that the previous behavior is "lying", the behavior identifying unit 104 determines whether or not the first minimum atmospheric pressure difference value has decreased from the second minimum atmospheric pressure difference value by a predetermined threshold value TH₁ (> 0) or greater (Step S45). In other words, the behavior identifying unit 104 determines whether or not the changed amount of the first minimum atmospheric pressure difference value with respect to the second minimum atmospheric pressure difference value is less than or equal to a predetermined threshold value TH₁' (<0). Note that the threshold value TH₁' is obtained by reversing the sign of the threshold value TH₁.

If having determined at Step S45 that the changed amount in the first minimum atmospheric pressure difference value with respect to the second minimum atmospheric pressure difference value is less than or equal to the predetermined threshold value TH₁', the behavior identifying unit 104 identifies the behavior as "standing" (Step S46). This is because the case where the changed amount in the first minimum atmospheric pressure difference value with respect to the second minimum atmospheric pressure difference value is less than or equal to the predetermined threshold value TH₁' is equivalent to the case where the altitude of the tag 20 attached to the cow has increased by the predetermined threshold value TH₁ or greater.

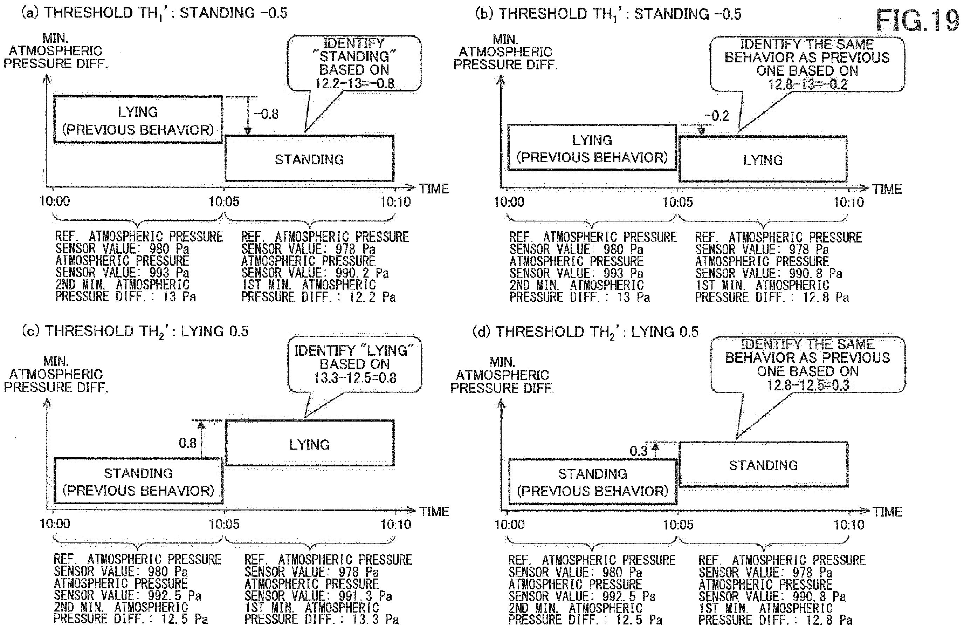

For example, as illustrated in FIG. 19(a), suppose that a second minimum atmospheric pressure difference between 10:00 and 10:05 is "13 Pa" (a reference atmospheric pressure sensor value of "980 Pa" and an atmospheric pressure sensor value of "993.0 Pa"), and a first minimum atmospheric pressure difference value between 10:05 and 10:10 is "12.2 Pa" (a reference atmospheric pressure sensor value of "978 Pa" and an atmospheric pressure sensor value of "990.2 Pa"). In this case, the changed amount "-0.8" of the first minimum atmospheric pressure difference value "12.2" with respect to the second minimum atmospheric pressure difference value "13" is less than or equal to a threshold value TH₁' "-0.5"; therefore, the behavior is identified as "standing".

On the other hand, if not having determined at Step S45 that the changed amount of the first minimum atmospheric pressure difference value with respect to the second minimum atmospheric pressure difference value is less than or equal to the predetermined threshold value TH₁', the behavior identifying unit 104 identifies the same behavior as the previous one (i.e., "lying") (Step S47). This is because the case where the changed amount in the first minimum atmospheric pressure difference value with respect to the second minimum atmospheric pressure difference value is not less than or equal to the predetermined threshold value TH₁' is equivalent to the case where the altitude of the tag 20 attached to the cow has not increased by the predetermined threshold value TH₁ or greater, and hence, the cow is considered to be continuously in a state of "lying".

For example, as illustrated in FIG. 19(b), assume that a second minimum atmospheric pressure difference value between 10:00 and 10:05 is "13 Pa" (a reference atmospheric pressure sensor value of "980 Pa" and an atmospheric pressure sensor value of "993 Pa"), and a first minimum atmospheric pressure difference value between 10:05 and 10:10 is "12.8 Pa" (a reference atmospheric pressure sensor value of "978 Pa" and an atmospheric pressure sensor value of "990.8 Pa"). In this case, the changed amount "-0.2" of the first minimum atmospheric pressure difference value "12.8" with respect to the second minimum atmospheric pressure difference value "13" is not less than or equal to the threshold value TH₁' "-0.5"; therefore, the behavior is identified as the same as the previous behavior of "lying".

If having determined at Step S44 that the previous behavior is other than "lying" (i.e., "standing"), the behavior identifying unit 104 determines whether or not the first minimum atmospheric pressure difference value has increased from the second minimum atmospheric pressure difference value by a predetermined threshold value TH₂ (<0) or greater (Step S48). In other words, the behavior identifying unit 104 determines whether or not the changed amount of the first minimum atmospheric pressure difference value with respect to the second minimum atmospheric pressure difference value is greater than or equal to a predetermined threshold value TH₂' (> 0). Note that the threshold value TH₂' is obtained by reversing the sign of the threshold value TH₂.

If having determined at Step S48 that the changed amount in the first minimum atmospheric pressure difference value with respect to the second minimum atmospheric pressure difference value is greater than or equal to the predetermined threshold value TH₂', the behavior identifying unit 104 identifies the behavior as "lying" (Step S49). This is because the case where the changed amount in the first minimum atmospheric pressure difference value with respect to the second minimum atmospheric pressure difference value is greater than or equal to the predetermined threshold value TH₂' is equivalent to the case where the altitude of the tag 20 attached to the cow has decreased by the predetermined threshold value TH₂' or greater.

For example, as illustrated in FIG. 19(c), suppose that a second minimum atmospheric pressure difference value between 10:00 and 10:05 is "12.5 Pa" (a reference atmospheric pressure sensor value of "980 Pa" and an atmospheric pressure sensor value of "992.5 Pa"), and a first minimum atmospheric pressure difference value between 10:05 and 10:10 is "13.3 Pa" (a reference atmospheric pressure sensor value of "978 Pa" and an atmospheric pressure sensor value of "991.3 Pa"). In this case, the changed amount "0.8" of the first minimum atmospheric pressure difference value "13.3" with respect to the second minimum atmospheric pressure difference value "12.5" is greater than or equal to the threshold value TH₂' "0.5"; therefore, the behavior is identified as "lying".

On the other hand, if not having determined at Step S48 that the changed amount of the first minimum atmospheric pressure difference value with respect to the second minimum atmospheric pressure difference value is not greater than or equal to the predetermined threshold value TH₂, the behavior identifying unit 104 identifies the same behavior as the previous one (i.e., "standing") (Step S50). This is because the case where the changed amount of the first minimum atmospheric pressure difference value with respect to the second minimum atmospheric pressure difference value is not greater than or equal to the predetermined threshold value TH₂' is equivalent to the case where the altitude of the tag 20 attached to the cow has not decreased by the predetermined threshold value TH₂', and hence, the cow is considered to be continuously in a state of "standing".

For example, as illustrated in FIG. 19(d), assume that a second minimum atmospheric pressure difference value between 10:00 and 10:05 is "12.5 Pa" (a reference atmospheric pressure sensor value of "980 Pa" and an atmospheric pressure sensor value of "992.5 Pa"), and a first minimum atmospheric pressure difference value between 10:05 and 10:10 is "12.8 Pa" (a reference atmospheric pressure sensor value of "978 Pa" and an atmospheric pressure sensor value of "990.8 Pa"). In this case, the changed amount "0.3" of the first minimum atmospheric pressure difference value "12.8" with respect to the second minimum atmospheric pressure difference value "12.5" is not greater than or equal to the predetermined threshold value TH₂' "0.5"; therefore, the behavior is identified as the same as the previous behavior of "standing".

As described above, the behavior identifying system 1 according to the present embodiment can identify a behavior of a cow from measured data received from a tag 20 attached to the cow. At this time, the behavior identifying system 1 according to the present embodiment identifies a behavior of the cow by using indicators calculated from acceleration sensor values included in the measured data and the behavior identifying model 300 generated in advance by a machine learning method (behavior identification by motion-activeness analysis). In particular, by using the standard deviation and the maximum value of L2 norms as the indicators, it is possible to identify "ruminating" as a behavior peculiar to cows with high accuracy. In other words, "ruminating" as a behavior peculiar to cows can be distinguished from behaviors such as "standing" and "lying" with high accuracy.

Also, in the case of having identified the behavior of a cow as "standing", "lying", or "ruminating" by behavior identification by the motion-activeness analysis, the behavior identifying system 1 according to the present embodiment identifies the behavior more precisely from atmospheric pressure sensor values and reference atmospheric pressure sensor values (behavior identification by atmospheric pressure analysis). This enables to identify "standing" (including "ruminating while standing") or "lying" (including "ruminating while lying") with high accuracy. Further, for example, even in a cowshed where there is a mound or the like such as a free-barn type cowshed, it is possible to identify "standing" (including "ruminating while standing") or "lying" (including "ruminating while lying") with high accuracy.

By displaying behaviors of cows identified in this way, for example, on a display device (a display or the like) of the behavior identifying device 10 to be presented to a dairy farmer or the like, it is possible to easily confirm behaviors often observed during the estrus. Also, this also leads to early detection of abnormal behaviors due to illness or injury, and makes health care for the cows easier.

### <Identification of behavior in free-barn type cowshed>

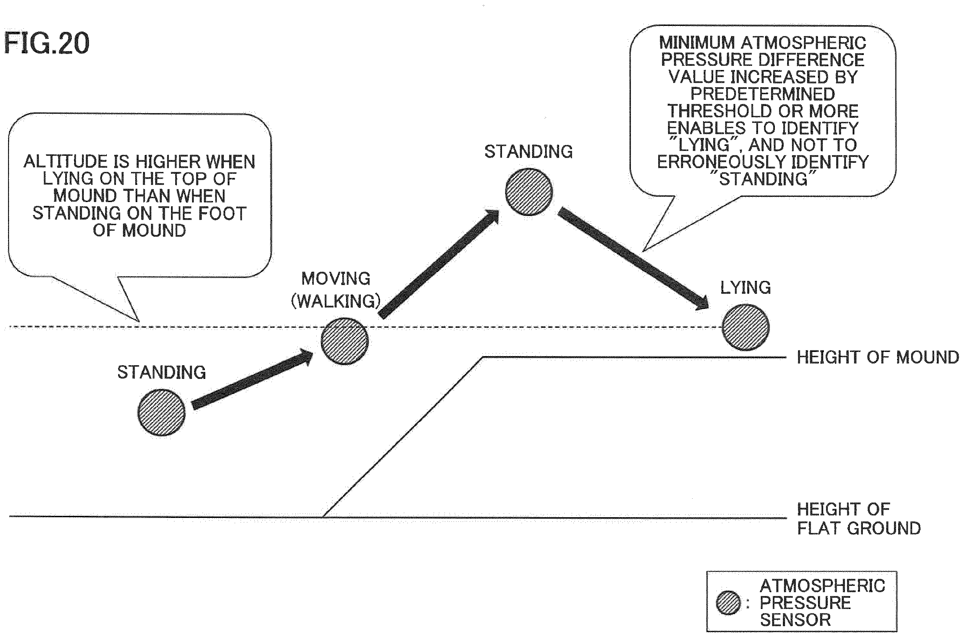

Here, a case of identifying a behavior in a free-barn type cowshed by the atmospheric pressure analysis according to the present embodiment will be described with reference to FIG. 20. FIG. 20 is a diagram illustrating an example in the case of identifying a behavior by the atmospheric pressure analysis in a free-barn type cowshed.

As illustrated in FIG. 20, in a free-barn type cowshed, there may be a mound in the cowshed. For this reason, the altitude of the tag 20 changes as the cow climbs up the mound. Even when the cow climbs up the mound, the atmospheric pressure analysis according to the present embodiment can identify one of the behavior of the cow among "standing" (including "ruminating while standing") or "lying" (including "ruminating while lying") with high accuracy.

For example, as illustrated in FIG. 20, consider a case where the altitude of the atmospheric pressure sensor when the cow is "lying" on the mound is higher than the altitude of the atmospheric pressure sensor when the cow is "standing" at the foot of the mound. At this time, for example, if the cow lies down after climbing the mound from the foot of the mound, the method of identifying the behavior of a cow by comparing atmospheric pressure sensor values with a predetermined threshold value may erroneously identify the behavior of the cow lying on the mound as "standing". In contrast, the atmospheric pressure analysis according to the present embodiment identifies a behavior of a cow based on whether a minimum atmospheric pressure difference value has increased (or decreased) by a predetermined threshold value or greater; therefore, if the cow lies down after climbing the mound from the foot of the mound, it is possible to correctly identify the behavior of the cow as "lying".

In this way, the behavior identifying system 1 according to the present embodiment can identify a behavior of a cow by atmospheric pressure analysis with high accuracy, for example, even if there is a mound in a cowshed as in a free-barn type cowshed, and the cow climbs the mound.

This application is based on a basic application 2017-070138 filed on March 31, 2017, in Japan and a basic application 2017-192742 filed on October 2, 2017, in Japan, and the entire contents of these applications are hereby incorporated by reference.

The present invention is not limited to the specifically disclosed embodiments, and various modifications and changes can be made without departing from the scope of the claims.

### LIST OF REFERENCE SYMBOLS

- 1: behavior identifying system
- 10: behavior identifying device
- 20: tag
- 30: leaky coaxial antenna
- 100: behavior identifying processor
- 101: obtainer
- 102: preprocessor
- 103: indicator calculator
- 104: behavior identifying unit
- 200: measured data memory unit
- 300: behavior identifying model

## Claims

1. A behavior identifying device that identifies a behavior of a farm animal, comprising:
a memory unit configured to store acceleration data measured by an acceleration sensor attached to the farm animal;
an obtainer configured to obtain one or more acceleration data items for a predetermined period of time from among the acceleration data stored in the memory unit;
an indicator calculator configured to calculate predetermined indicators from the one or more acceleration data items obtained by the obtainer; and
an identifying unit configured to identify the behavior of the farm animal based on the indicators calculated by the indicator calculator and an identification model generated in advance for identifying the behavior of the farm animal.

2. The behavior identifying device as claimed in claim 1, wherein the indicator calculator calculates a standard deviation and a maximum value of the one or more acceleration data items as the indicators.

3. The behavior identifying device as claimed in claim 1 or 2, wherein the memory unit further stores radio wave strength data measured by a radio wave sensor attached to the farm animal, and
wherein in a case where the identified behavior of the farm animal is a predetermined behavior, the identifying unit identifies a detailed behavior of the farm animal based on the radio wave strength data.

4. The behavior identifying device as claimed in any one of claims 1 to 3, wherein the behavior includes a standing behavior representing that the farm animal is standing and a lying behavior representing that the farm animal is lying,
wherein the memory unit further stores atmospheric pressure data measured by the atmospheric pressure sensor attached to the farm animal, and
wherein the identifying unit identifies the standing behavior or the lying behavior of the farm animal based on the atmospheric pressure data.

5. The behavior identifying device as claimed in claim 4, wherein the identifying unit identifies the standing behavior or the lying behavior of the farm animal, based on a changing pattern of the acceleration data or a changing pattern of the atmospheric pressure data.

6. A behavior identifying device that identifies a behavior of a farm animal, comprising:
a memory unit configured to store acceleration data measured by an acceleration sensor, and atmospheric pressure data measured by an atmospheric pressure sensor, wherein the acceleration sensor and the atmospheric pressure sensor are attached to the farm animal;
an obtainer configured to obtain one or more acceleration data items and one or more atmospheric pressure data items for a predetermined period of time from the memory unit;
an indicator calculator configured to calculate predetermined indicators from the one or more acceleration data items obtained by the obtainer;
a first identifying unit configured to identify the behavior of the farm animal based on the indicators calculated by the indicator calculator and an identification model generated in advance for identifying the behavior of the farm animal; and
a second identifying unit configured to identify, in a case where the behavior of the farm animal identified by the first identifying unit is a predetermined behavior, further identify the behavior based on the one or more atmospheric pressure data items obtained by the obtainer.

7. The behavior identifying device as claimed in claim 6, wherein in a case where the behavior identified by the first identifying unit is one of a standing behavior representing that the farm animal is standing, a lying behavior representing that the farm animal is lying, and a ruminating behavior representing that the farm animal is ruminating, the second identifying unit identifies the behavior of the farm animal based on the one or more atmospheric pressure data items obtained by the obtainer.

8. The behavior identifying device as claimed in claim 6 or 7, wherein the second identifying unit calculates a first value representing a difference between an atmospheric pressure value represented by an atmospheric pressure data item and a reference atmospheric pressure value corresponding to the atmospheric pressure value within a first predetermined period of time from among the one or more atmospheric pressure data items, and
calculates a second value representing a difference between an atmospheric pressure value represented by an atmospheric pressure data item and a reference atmospheric pressure value corresponding to the atmospheric pressure value within a second predetermined period of time representing a period of time prior to the first predetermined period of time, from among the one or more atmospheric pressure data items,
to identify the behavior of the farm animal according to a comparison between the first value and the second value that have been calculated.

9. The behavior identifying device as claimed in claim 8, wherein the second identifying unit identifies the behavior of the farm animal as the lying behavior in a case where a behavior of the farm animal at a previous time is other than the lying behavior, and the first value has increased from the second value by a predetermined threshold value or greater, and
identifies the behavior of the farm animal as the standing behavior in a case where the behavior of the farm animal at the previous time is the lying behavior, and the first value has decreased from the second value by a predetermined threshold value or greater.

10. The behavior identifying device as claimed in claim 8 or 9, wherein the second identifying unit identifies, in a case where the behavior of the farm animal at a previous point in time is other than the lying behavior and the first value has not increased from the second value by the predetermined threshold value or greater, the behavior of the farm animal as a same behavior of the farm animal at the previous point in time, and
identifies, in a case where the behavior of the farm animal at the previous point in time is the lying behavior and the first value has not decreased from the second value by the predetermined threshold value or greater, the behavior of the farm animal as the same behavior of the farm animal at the previous point in time.

11. The behavior identifying device as claimed in any one of claims 8 to 10, wherein the first value is a minimum value, an average value, or a maximum value of differences between atmospheric pressure values represented by atmospheric pressure data items and reference atmospheric pressure values corresponding to the atmospheric pressure values, respectively, for the first predetermined period of time, from among the one or more atmospheric pressure data items, and
the second value is a minimum value, an average value, or a maximum value of differences between atmospheric pressure values represented by atmospheric pressure data items and reference atmospheric pressure values corresponding to the atmospheric pressure values, respectively, for the second predetermined period of time, from among the one or more atmospheric pressure data items.

12. The behavior identifying device as claimed in any one of claims 1 to 11, wherein the farm animal is a cow.

13. A behavior identifying method executed by a behavior identifying device that identifies a behavior of a farm animal, and includes a memory unit storing acceleration data measured by an acceleration sensor attached to the farm animal, the method comprising:
an obtaining step of obtaining one or more acceleration data items for a predetermined period of time from among the acceleration data stored in the memory unit;
an indicator calculating step of calculating predetermined indicators from the one or more acceleration data items obtained by the obtaining step; and
an identifying step for identifying the behavior of the farm animal based on the indicators calculated by the indicator calculation step and an identification model generated in advance for identifying the behavior of the farm animal.

14. A behavior identifying method executed by a behavior identifying device that identifies a behavior of a farm animal, and includes a memory unit storing acceleration data measured by an acceleration sensor and atmospheric pressure data measured by an atmospheric pressure sensor attached to the farm animal, wherein the acceleration sensor and the atmospheric pressure sensor are attached to the farm animal, the method comprising:
an obtaining step of obtaining one or more acceleration data items and one or more atmospheric pressure data items for a predetermined period of time from the memory unit;
an indicator calculating step of calculating predetermined indicators from the one or more acceleration data items obtained by the obtaining step;
a first identifying step of identifying the behavior of the farm animal based on the indicators calculated by the indicator calculation step and an identification model generated in advance for identifying the behavior of the farm animal; and
a second identifying step for identifying, in a case where the behavior identified by the first identifying step is a predetermined behavior, the behavior of the farm animal based on the one or more atmospheric pressure data items obtained by the obtainment step.

15. A program causing a computer to function as respective units in the behavior identifying device as claimed in any one of claims 1 to 12.
